(19) Europäisches Patentamt
European Patent Office
Office européen des brevets

(11) EP 3 215 227 B1

(12) EUROPÄISCHE PATENTSCHRIFT

(45) Veröffentlichungstag und Bekanntmachung des Hinweises auf die Patenterteilung:
**13.03.2019 Patentblatt 2019/11**

(21) Anmeldenummer: **15759476.3**

(22) Anmeldetag: **09.09.2015**

(51) Int Cl.:
***A61Q 5/10*** (2006.01) ***A61K 8/58*** (2006.01)
***A61K 8/86*** (2006.01) ***A61K 8/891*** (2006.01)
***A61K 8/898*** (2006.01)

(86) Internationale Anmeldenummer:
**PCT/EP2015/070548**

(87) Internationale Veröffentlichungsnummer:
**WO 2016/071024 (12.05.2016 Gazette 2016/19)**

# (54) OXIDATIONSFÄRBEMITTEL, ENTHALTEND EINE KOMBINATION AUS VERNETZTEN, AMINIERTEN SILOXANPOLYMEREN UND NICHTIONISCHEN TENSIDEN

OXIDATIVE HAIR DYE COMPOSITION COMPRISING A COMBINATION OF CROSS LINKED AMINATED SILOXANPOLYMERS AND NON IONIC TENSIDS

COMPOSITION POUR LA COLORATION DES FIBRES CERATINIQUES COMPRENANT DES AMINOSILOXANES ET DES TENSIOACTIVES NON-IONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.11.2014 DE 102014222374**

(43) Veröffentlichungstag der Anmeldung:
**13.09.2017 Patentblatt 2017/37**

(73) Patentinhaber: **Henkel AG & Co. KGaA**
**40589 Düsseldorf (DE)**

(72) Erfinder:
- **KERL, Sylvia**
**22763 Hamburg (DE)**
- **HAGENOW, Susanne**
**20359 Hamburg (DE)**

(56) Entgegenhaltungen:
**EP-A1- 1 921 106**
**WO-A2-2014/095178**
**DE-A1-102009 027 678**
**US-A1- 2014 161 756**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 215 227 B1

## Beschreibung

Die vorliegende Erfindung betrifft kosmetische Mittel zur Färbung keratinischer Fasern, welche eine Kombination von vernetzten, aminierten Siloxanpolymeren und nichtionischen Tensiden enthalten.

Weiterhin betrifft die vorliegende Erfindung eine Verpackungseinheit (Kit-of-parts), enthaltend ein erfindungsgemäßes kosmetisches Mittel sowie eine Oxidationsmittelzubereitung.

Darüber hinaus betrifft die vorliegende Erfindung ein Verfahren zum Färben von keratinischen Fasern unter Verwendung eines erfindungsgemäßen kosmetischen Mittels sowie einer Oxidationsmittelzubereitung.

Zudem betrifft die vorliegende Erfindung die Verwendung eines erfindungsgemäßen kosmetischen Mittels zur Erhöhung der Farbtiefe.

Schließlich betrifft die vorliegende Erfindung die Verwendung einer erfindungsgemäßen Verpackungseinheit zur Herstellung eines Oxidationsfärbemittels zur Färbung keratinischer Fasern mit erhöhter Farbtiefe.

Die Veränderung der Form und der Farbe von Haaren stellt einen wichtigen Bereich der modernen Kosmetik dar. Dadurch kann das Erscheinungsbild der Haare sowohl an aktuelle Modeströmungen als auch an die individuellen Wünsche des einzelnen Verbrauchers angepasst werden. Die modische Farbgestaltung von Frisuren oder die Kaschierung von ergrautem oder weißem Haar mit modischen oder natürlichen Farbtönen erfolgt üblicherweise mit farbverändernden Mitteln. Diese Mittel sollen neben einer hohen Färbeleistung zusätzliche Eigenschaften, wie beispielsweise die Steigerung des Haarvolumens, aufweisen.

Zur Bereitstellung farbverändernder kosmetischer Mittel, insbesondere für die Haut oder für keratinhaltige Fasern, wie beispielsweise menschliche Haare, sind im Stand der Technik verschiedene Färbesysteme bekannt.

Für permanente, intensive Färbungen mit entsprechenden Echtheitseigenschaften werden Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehrerer Kupplerkomponenten die eigentlichen Farbstoffe aus. Die Oxidationsfärbemittel zeichnen sich durch hervorragende, lang anhaltende Färbeergebnisse aus. Für natürlich wirkende Färbungen muss üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Für temporäre Färbungen werden üblicherweise Färbe- oder Tönungsmittel verwendet, welche als färbende Komponente sogenannte Direktzieher enthalten. Hierbei handelt es sich um Farbstoffmoleküle, welche direkt auf die keratinischen Fasern aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als oxidativen Färbungen, so dass sehr viel früher eine vielfach unerwünschte Nuancenverschiebung oder ein sichtbarer homogener Farbverlust eintritt.

Schließlich hat ein weiteres Färbeverfahren große Beachtung gefunden. Bei diesem Verfahren werden Vorstufen des natürlichen Haarfarbstoffes Melanin auf die z.B. Haare aufgebracht; diese bilden dann im Rahmen oxidativer Prozesse im Haar naturanaloge Farbstoffe aus. In solchen Verfahren wird beispielsweise 5,6-Dihydroxyindolin als Farbstoffvorprodukt eingesetzt. Insbesondere bei mehrfacher Anwendung von Mitteln mit 5,6-Dihydroxyindolin ist es möglich, bei Personen mit ergrauten Haaren die natürliche Haarfarbe wiederherzustellen. Die Ausfärbung kann dabei mit Luftsauerstoff als einzigem Oxidationsmittel erfolgen, so dass auf weitere Oxidationsmittel verzichtet werden kann. Bei Personen mit ursprünglich mittelblondem bis braunem Haar kann das 5,6-Dihydroxyindolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden.

Die im Stand der Technik bekannten oxidativen Färbemittel führen jedoch nicht immer zu der gewünschten hohen Färbeleistung, insbesondere zu einer hohen Farbtiefe.

Der vorliegenden Erfindung lag daher die Aufgabe zugrunde, ein kosmetisches Mittel zur Färbung keratinischer Fasern bereitzustellen, welches die Nachteile des Standes der Technik vermeidet bzw. zumindest abschwächt und welches in einer verbesserten Farbtiefe resultiert.

Es wurde nun überraschenderweise gefunden, dass der Zusatz einer Kombination aus einem vernetzten, aminierten Siloxanpolymer und nichtionischen Tensiden in kosmetischen Mitteln zur Färbung keratinischer Fasern, insbesondere menschlicher Haare, zu einer Erhöhung der Farbtiefe führt. Im Vergleich zu Färbemitteln des Standes der Technik, welche keine Kombination aus einem vernetzten, aminierten Siloxanpolymer und nichtionischen Tensiden enthalten, kann daher zur Erreichung einer vergleichbaren Farbtiefe eine signifikant reduzierte Gesamtmenge an Farbstoffen in den erfindungsgemäßen kosmetischen Mitteln eingesetzt werden. Darüber hinaus führt der Einsatz der zuvor genannten Kombination nicht zu negativen Wechselwirkungen mit den weiteren Inhaltsstoffen der erfindungsgemäßen kosmetischen Mittel, so dass eine hohe Lagerstabilität gewährleistet ist.

Ein erster Gegenstand der Erfindung ist daher ein kosmetisches Mittel zur Farbveränderung keratinischer Fasern, umfassend in einem kosmetisch verträglichen Träger

a) mindestens eine Verbindung, ausgewählt aus der Gruppe von Oxidationsfarbstoffvorprodukten, direktziehenden Farbstoffen sowie deren Mischungen,

b) mindestens ein vernetztes, aminiertes Siloxanpolymer, welches durch Umsetzung eines Copolymers, bestehend aus 3-(2-Aminoethylamino)propylmethylsiloxyeinheiten und Dimethylsiloxyeinheiten, mit N-Morpholinomethyltriethoxysilan erhältlich ist, in einer Gesamtmenge von 0,3 bis 1,0 Gew.-%, bezogen auf das Gesamtgewicht des kosmetischen Mittels, und

c) mindestens ein nichtionisches Tensid der Formel (I)

$$R\text{-}O\text{-}(CH_2\text{-}CH_2\text{-}O)_n\text{-}H \qquad (I),$$

worin

R für eine lineare oder verzweigte Alkylkette mit 12 bis 16 Kohlenstoffatomen steht, und
n für ganze Zahlen von 6 bis 15 steht.

Unter dem Begriff "keratinische Fasern oder auch Keratinfasern" sind erfindungsgemäß Pelze, Wolle, Federn sowie menschliche Haare zu verstehen. Im Rahmen der vorliegenden Erfindung ist es besonders bevorzugt, wenn die kosmetischen Mittel zur Färbung von menschlichen Haaren verwendet werden.

Weiterhin werden unter dem Begriff "vernetzte, aminierte Siloxanpolymere" im Rahmen der vorliegenden Erfindung Polymere verstanden, in welchen die Siliciumatome über Sauerstoffatome miteinander verbunden sind, d.h. in welchen Si-O-Si-Bindungen vorliegen, und welche mindestens eine Amingruppe aufweisen. Unter "vernetzt" bzw. "Vernetzung" ist im Sinne der Erfindung die Verknüpfung von Polymerketten des Siloxanpolymers miteinander durch kovalente chemische Bindung unter Bildung eines Netzwerkes zu verstehen. Diese kovalente Verknüpfung der Polymerketten des Siloxanpolymers kann entweder mittels direkter kovalenter Bindung erfolgen oder durch ein die Polymerketten verbrückendes Molekülfragment vermittelt werden. Das Molekülfragment bindet an die durch das Molekülfragment verbrückten Polymerketten des Siloxanpolymers jeweils mittels kovalenter chemischer Bindung.

Zudem werden unter dem Begriff "nichtionische Tenside" amphiphile (bifunktionelle) Verbindungen verstanden, welche aus mindestens einem hydrophoben und mindestens einem hydrophilen Molekülteil bestehen und welche keine kationisierbaren und/oder anionisierbaren Gruppen, wie beispielsweise Amingruppen, Carboxylatgruppen, Sulfatgruppen oder dergleichen, aufweisen. Die nichtionischen Tenside im Rahmen der vorliegenden Erfindung zeigen eine orientierte Absorption an Grenzflächen sowie eine Aggregation zu Mizellen und die Ausbildung von lyotrophen Phasen.

Darüber hinaus werden unter dem Begriff "Fettalkohole" im Rahmen der vorliegenden Erfindung aliphatische, langkettige, einwertige, primäre Alkohole verstanden, welche unverzweigte Kohlenwasserstoffreste mit 6 bis 30 Kohlenstoffatomen aufweisen. Die Kohlenwasserstoffreste können gesättigt aber auch ein- oder mehrfach ungesättigt sein.

Schließlich werden unter dem Begriff "Fettsäuren" im Rahmen der vorliegenden Erfindung aliphatische Monocarbonsäuren mit unverzweigter Kohlenstoffkette verstanden, welche Kohlenwasserstoffreste mit 6 bis 30 Kohlenstoffatomen aufweisen. Die Kohlenwasserstoffreste können entweder gesättigt oder auch ein- oder mehrfach ungesättigt sein.

Die Angabe der Gesamtmenge in Bezug auf die Komponenten des kosmetischen Mittels bezieht sich vorliegend - sofern nichts anderes angegeben ist - auf die Gesamtmenge an Aktivsubstanz der jeweiligen Komponente. Weiterhin bezieht sich die Angabe der Gesamtmenge in Bezug auf die Komponenten des kosmetischen Mittels - sofern nichts anderes angegeben ist - auf das Gesamtgewicht des oxidationsmittelfreien erfindungsgemäßen kosmetischen Mittels.

Die erfindungsgemäßen Mittel enthalten einen kosmetischen Träger. Erfindungsgemäß bevorzugt ist der kosmetische Träger wässrig, alkoholisch oder wässrig-alkoholisch. Im Rahmen der vorliegenden Erfindung können beispielsweise Cremes, Emulsionen, Gele oder tensidhaltige schäumende Lösungen, wie beispielsweise Shampoos, Schaumaerosole oder andere Zubereitungen, welche für die Anwendung auf dem Haar geeignet sind, eingesetzt werden.

Ein wässriger Träger enthält im Sinne der Erfindung enthält, bezogen auf das Gesamtgewicht des kosmetischen Mittels, mindestens 30 Gew.-%, insbesondere mindestens 50 Gew.-%, Wasser.

Unter wässrig-alkoholischen Trägern sind im Sinne der vorliegenden Erfindung wasserhaltige Zusammensetzungen, enthaltend einen $C_1$-$C_4$-Alkohol in einer Gesamtmenge von 3 bis 90 Gew.-%, bezogen auf das Gesamtgewicht des kosmetischen Mittels, insbesondere Ethanol bzw. Isopropanol, zu verstehen.

Die erfindungsgemäßen Mittel können zusätzlich weitere organische Lösungsmittel, wie beispielsweise Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel, wobei das Lösungsmittel in einer Gesamtmenge von 0,1 bis 30 Gew.-%, vorzugsweise von 1 bis 20 Gew.-%, insbesondere von 2 bis 10 Gew.-%, bezogen auf das Gesamtgewicht des kosmetischen Mittels, enthalten ist.

Das erfindungsgemäße kosmetische Mittel enthält als ersten wesentlichen Bestandteil a) eine Verbindung, ausgewählt aus der Gruppe von Oxidationsfarbstoffvorprodukten (OFV), direktziehenden Farbstoffe (DZ) sowie deren Mischungen.

In einer bevorzugten Ausführungsform enthalten erfindungsgemäße Mittel mindestens ein Oxidationsfarbstoffvorprodukt.

Oxidationsfarbstoffvorprodukte können aufgrund ihres Reaktionsverhaltens in zwei Kategorien eingeteilt werden, sogenannte Entwicklerkomponenten und Kupplerkomponenten. Entwicklerkomponenten können mit sich selbst den eigentlichen Farbstoff ausbilden. Sie können daher als alleinige Verbindungen im erfindungsgemäßen kosmetischen Mittel enthalten sein. In einer bevorzugten Ausführungsform enthalten die erfindungsgemäßen kosmetischen Mittel daher mindestens ein Oxidationsfarbstoffvorprodukt vom Entwicklertyp. Es kann im Rahmen der vorliegenden Erfindung jedoch auch vorgesehen sein, dass die erfindungsgemäßen kosmetischen Mittel mindestens ein Oxidationsfarbstoffvorprodukt vom Kupplertyp enthalten. Besonders gute Ergebnisse in Bezug auf die Färbung keratinischer Fasern werden erhalten, wenn die erfindungsgemäßen kosmetischen Mittel mindestens ein Oxidationsfarbstoffvorprodukt vom Entwicklertyp und mindestens ein Oxidationsfarbstoffvorprodukt vom Kupplertyp enthalten.

Die Entwickler- und Kupplerkomponenten werden üblicherweise in freier Form eingesetzt. Bei Substanzen mit Aminogruppen kann es jedoch bevorzugt sein, deren Salzform, insbesondere in Form der Hydrochloride und Hydrobromide oder der Sulfate, einzusetzen.

Erfindungsgemäß sind kosmetische Mittel bevorzugt, welche die Entwickler- und/oder Kupplerkomponenten jeweils in einer Gesamtmenge von 0,001 bis 10 Gew.-%, vorzugsweise von 0,01 bis 8 Gew.-%, bevorzugt von 0,1 bis 5 Gew.-%, insbesondere von 0,5 bis 3 Gew.-%, bezogen auf das Gesamtgewicht des kosmetischen Mittels, enthalten.

In einer weiteren bevorzugten Ausführungsform ist das erfindungsgemäße kosmetische Mittel daher dadurch gekennzeichnet, dass es ein Oxidationsfarbstoffvorprodukt vom Entwickler und/oder Kupplertyp in einer Gesamtmenge von 0,001 bis 5,0 Gew.-%, vorzugsweise von 0,01 bis 4,0 Gew.-%, bevorzugt von 0,1 bis 3,0 Gew.-%, insbesondere von 0,5 bis 2,0 Gew.-%, bezogen auf das Gesamtgewicht des kosmetischen Mittels, enthält.

Geeignete Oxidationsfarbstoffvorprodukte vom Entwicklertyp sind beispielsweise p-Phenylendiamin und dessen Derivate. Bevorzugte p-Phenylendiamine werden ausgewählt aus einer oder mehrerer Verbindungen der Gruppe, welche gebildet wird aus p-Phenylendiamin, p-Toluylendiamin, 2-Chlor-p-phenylendiamin, 2,3-Dimethyl-p-phenylendiamin, 2,6-Dimethyl-p-phenylendiamin, N,N-Bis-(2-hydroxyethyl)-p-phenylen-diamin, 2-(2-Hydroxyethyl)-p-phenylendiamin, 2-(1,2-Dihydroxyethyl)-p-phenylendiamin, N-(2-Hydroxypropyl)-p-phenylendiamin, N-(4'-Aminophenyl)-p-phenylendiamin, 2-Methoxymethyl-p-phenylendiamin, N-Phenyl-p-phenylendiamin, 2-(2-Hydroxyethyloxy)-p-phenylendiamin und N-(4-Amino-3-methyl-phenyl)-N-[3-(1H-imidazol-1-yl)propyl]amin sowie ihren physiologisch verträglichen Salzen.

Es kann erfindungsgemäß weiterhin bevorzugt sein, als Entwicklerkomponente Verbindungen einzusetzen, welche mindestens zwei aromatische Kerne enthalten, die mit Amino- und/oder Hydroxylgruppen substituiert sind. Bevorzugte zweikernige Entwicklerkomponenten werden ausgewählt aus N,N'-Bis-(2-hydroxyethyl)-N,N'-bis-(4'-aminophenyl)-1,3-diaminopropan-2-ol, N,N'-Bis-(4-aminophenyl)-1,4-diazacycloheptan, Bis-(2-hydroxy-5-aminophenyl)methan sowie deren physiologisch verträglichen Salzen.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind p-Aminophenol, N-Methyl-p-Aminophenol, 4-Amino-3-methylphenol, 4-Amino-2-aminomethyl-phenol, 4-Amino-2-(1,2-dihydroxyethyl)phenol, 4-Amino-2-(diethylaminomethyl)phenol sowie deren physiologisch verträglichen Salze.

Ferner kann die Entwicklerkomponente ausgewählt sein aus o-Aminophenol und dessen Derivaten, bevorzugt aus 2-Amino-4-methylphenol, 2-Amino-5-methylphenol, 2-Amino-4-chlorphenol und/oder deren physiologisch verträglichen Salzen.

Weiterhin kann die Entwicklerkomponente ausgewählt sein aus heterocyclischen Entwicklerkomponenten, wie Pyrimidin-Derivaten, Pyrazol-Derivaten, Pyrazolopyrimidin-Derivaten bzw. ihren physiologisch verträglichen Salzen. Bevorzugte Pyrimidin-Derivate sind 2,4,5,6-Tetraaminopyrimidin, 4-Hydroxy-2,5,6-triaminopyrimidin und deren physiologisch verträgliche Salze. Ein bevorzugtes Pyrazol-Derivat ist 4,5-Diamino-1-(2-hydroxyethyl)pyrazol sowie dessen physiologisch verträglichen Salze. Als Pyrazolopyrimidine sind insbesondere Pyrazolo[1,5-a]pyrimidine bevorzugt.

Bevorzugte Oxidationsfarbstoffvorprodukte vom Entwicklertyp sind ausgewählt aus der Gruppe, welche gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-amino-methylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin oder den physiologisch verträglichen Salzen dieser Verbindungen.

Besonders bevorzugte Entwicklerkomponenten sind p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)-propyl]amin, und/oder 4,5-Diamino-1-(2-hydroxyethyl)pyrazol sowie deren physiologisch verträglichen Salze.

Gemäß einer weiteren bevorzugten Ausführungsform der vorliegenden Erfindung enthält das erfindungsgemäße kosmetische Mittel als Oxidationsfarbstoffvorprodukt neben mindestens einer Entwicklerkomponente weiterhin zusätzlich mindestens eine Kupplerkomponente. Als Kupplerkomponenten werden in der Regel m-Phenylendiaminderivate, Naphthole, Resorcin und Resorcinderivate, Pyrazolone und m-Aminophenolderivate verwendet.

Erfindungsgemäß bevorzugte Kupplerkomponenten sind ausgewählt aus

(A) m-Aminophenol und dessen Derivaten, insbesondere 3-Aminophenol, 5-Amino-2-methylphenol, 3-Amino-2-chlor-6-methylphenol, 5-Amino-4-chlor-2-methylphenol, 5-(2'-Hydroxyethyl)-amino-2-methylphenol und 2,4-Dichlor-3-aminophenol,
(B) o-Aminophenol und dessen Derivaten, wie 2-Amino-5-ethylphenol,
(C) m-Diaminobenzol und dessen Derivaten, wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 2,6-Bis-(2'-hydroxyethylamino)-1-methylbenzol, 2-({3-[(2-Hydroxyethyl)amino]-4-methoxy-5-methylphenyl}amino)ethanol und 2-({3-[(2-Hydroxyethyl)amino]-2-methoxy-5-methylphenyl}-amino)ethanol,
(D) o-Diaminobenzol und dessen Derivaten,
(E) Di- beziehungsweise Trihydroxybenzolderivaten, insbesondere Resorcin, 2-Chlorresorcin, 4-Chlorresorcin, 2-Methylresorcin und 1,2,4-Trihydroxybenzol,
(F) Pyridinderivaten, insbesondere 3-Amino-2-methylamino-6-methoxypyridin, 2,6-Diaminopyridin, 2,6-Dihydroxy-3,4-dimethylpyridin, 2-Amino-3-hydroxypyridin und 3,5-Diamino-2,6-dimethoxy-pyridin,
(G) Naphthalinderivaten, wie 1-Naphthol und 2-Methyl-1-naphthol,
(H) Morpholinderivaten, wie 6-Hydroxybenzomorpholin,
(I) Chinoxalinderivaten,
(J) Pyrazolderivaten, wie 1-Phenyl-3-methylpyrazol-5-on,
(K) Indolderivaten, wie 6-Hydroxyindol,
(L) Pyrimidinderivaten oder
(M) Methylendioxybenzolderivaten, wie 1-(2'-Hydroxyethyl)-amino-3,4-methylendioxybenzol

sowie deren physiologisch verträglichen Salze.

Erfindungsgemäß bevorzugte Kupplerkomponenten sind ausgewählt aus der Gruppe, welche gebildet wird aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-di-aminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder den physiologisch verträglichen Salzen der vorgenannten Verbindungen.

Erfindungsgemäß besonders bevorzugte Kupplerkomponenten sind Resorcin, 2-Methylresorcin, 5-Amino-2-methylphenol, 3-Aminophenol, 2-(2,4-Diaminophenoxy)ethanol, 1,3-Bis-(2,4-diaminophenoxy)propan, 1-Methoxy-2-amino-4-(2'-hydroxyethylamino)benzol, 2-Amino-3-hydroxypyridin, 2,6-Dihydroxy-3,4-dimethylpyridin, 1,5-Dihydroxynaphthalin, 2,7-Dihydroxynaphthalin und 1-Naphthol sowie deren physiologisch verträgliche Salze.

In einer bevorzugten Ausführungsform der vorliegenden Erfindung sind die erfindungsgemäßen kosmetischen Mittel dadurch gekennzeichnet, dass sie als Oxidationsfarbstoffvorprodukt mindestens eine Entwicklerkomponente, ausgewählt aus der Gruppe von p-Phenylendiamin, p-Toluylendiamin, N,N-Bis-(2-hydroxyethyl)amino-p-phenylendiamin, 1,3-Bis-[(2-hydroxyethyl-4'-aminophenyl)amino]-propan-2-ol, 1,10-Bis-(2',5'-diaminophenyl)-1,4,7,10-tetraoxadecan, 4-Aminophenol, 4- Amino-3-methylphenol, Bis-(5- amino-2-hydroxyphenyl)methan, 2,4,5,6- Tetraaminopyrimidin, 2-Hydroxy-4,5,6-triaminopyrimidin, 4,5-Diamino-1-(2-hydroxyethyl)-pyrazol, deren physiologisch verträglichen Salze sowie deren Mischungen, und mindestens eine Kupplerkomponente, ausgewählt aus der Gruppe von Resorcin, 2-Methylresorcin, 5-Methyl-resorcin, 2,5-Dimethylresorcin, 4-Chlorresorcin, Resorcinmonomethylether, 5-Aminophenol, 5-Amino-2-methylphenol, 5-(2-Hydroxyethyl)amino-2-methylphenol, 3- Amino-4-chlor-2-methylphenol, 3-Amino-2-chlor-6-methylphenol, 3-Amino-2,4-Dichlorphenol, 2,4-Diaminophenoxyethanol, 2-Amino-4-(2'-hydroxyethyl)amino-anisolsulfat, 1,3-Bis-(2,4-diaminophenoxy)propan, 2-Amino-3-hydroxypyridin, 2-Methylamino-3-amino-6-methoxypyridin, 2,6-Dihydroxy-3,4-dimethylpyridin, 3,5-Diamino-2,6-dimethoxypyridin, 1-Naphthol, 2-Methyl-1-naphthol, 1,5-Dihydroxynaphthalin, 2,7-Dihydroxynaphthalin, 1-Phenyl-3-methylpyrazol-5-on, 2,6-Bis-[(2'-hydroxyethyl)amino]-toluol, 4-Hydroxyindol, 6-Hydroxyindol, 6-Hydroxybenzomorpholin, deren physiologisch verträglichen Salze sowie deren Mischungen, enthalten.

Besonders bevorzugt im Rahmen der vorliegenden Erfindung sind kosmetische Mittel, welche als Oxidationsfarbstoffvorprodukt mindestens eine Entwicklerkomponente aus der Gruppe von p-Phenylendiaminen, insbesondere p-Toluylendiamin, und mindestens eine Kupplerkomponente aus der Gruppe von Resorcin, Resorcinderivaten, insbesondere 4-Chlorresorcin, und m-Aminophenolen, insbesondere 3-Aminophenol, sowie deren Salze und Mischungen, enthalten.

Um eine ausgewogene und subtile Nuancenausbildung zu erhalten, kann es im Rahmen der vorliegenden Erfindung auch vorgesehen sein, dass die erfindungsgemäßen kosmetischen Mittel zusätzlich mindestens einen direktziehenden Farbstoff enthalten. Bei direktziehenden Farbstoffen handelt sich um Farbstoffe, welche direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Direktziehende Farbstoffe können in anionische, kationische und nichtionische direktziehende Farbstoffe unterteilt werden.

Bevorzugte anionische direktziehende Farbstoffe sind die unter den Bezeichnungen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52 und Tetrabromphenolblau bekannten Verbindungen. Bevorzugte kationische direktziehende Farbstoffe sind kationische Triphenylmethanfarbstoffe, wie Basic Blue 7, Basic Blue 26, Basic Violet 2 und Basic Violet 14 sowie aromatische Systeme, welche mit einer quaternären Stickstoffgruppe substituiert sind, wie Basic Yellow 57, Basic Red 76, Basic Blue 99, Basic Brown 16 und Basic Brown 17 und HC Blue 16, sowie Basic Yellow 87, Basic Orange 31 und Basic Red 51. Bevorzugte nichtionische direktziehende Farbstoffe sind HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Walnuss, Faulbaumrinde, Salbei, Blauholz, Krappwurzel, Catechu und Alkannawurzel enthalten sind.

Bevorzugt enthält das erfindungsgemäße kosmetische Mittel die direktziehenden Farbstoffe in einer Gesamtmenge von 0,001 bis 10 Gew.-%, vorzugsweise von 0,01 bis 8 Gew.-%, bevorzugt von 0,1 bis 5 Gew.-%, insbesondere von 0,5 bis 3 Gew.-%, bezogen auf das Gesamtgewicht des kosmetischen Mittels.

Als zweiten wesentlichen Bestandteil b) enthalten die erfindungsgemäßen kosmetischen Mittel mindestens ein vernetztes, aminiertes Siloxanpolymer. Der Zusatz dieses Siloxanpolymers führt in Kombination mit dem nichtionischen Tensid zu einer Erhöhung der Farbtiefe und erlaubt daher eine Verringerung der Farbstoffmenge. Weiterhin sind die vernetzten, aminierten Siloxanpolymere gegenüber den zur Färbung verwendeten Oxidationsmitteln stabil und führen nicht zu negativen Wechselwirkungen mit den weiteren Inhaltsstoffen des erfindungsgemäßen kosmetischen Mittels, so dass eine hohe Lagerstabilität gewährleistet ist.

Im Rahmen der Erfindung werden bevorzugt vernetzte, aminierte Siloxanpolymere eingesetzt, welche durch Umsetzung von bestimmten Mengen des Copolymers, bestehend aus 3-(2-Aminoethylamino)propylmethylsiloxyeinheiten und Dimethylsiloxyeinheiten, mit bestimmten Mengen an N-Morpholinomethyltriethoxysilan erhältlich sind. Besonders bevorzugte Ausführungsformen der vorliegenden Erfindung sind daher dadurch gekennzeichnet, dass das mindestens eine vernetzte, aminierte Siloxanpolymer b) durch die Umsetzung eines Copolymers, bestehend aus 3-(2-Aminoethylamino)propylmethylsiloxyeinheiten und Dimethylsiloxyeinheiten, mit N-Morpholinomethyltriethoxysilan in einem Gewichtsverhältnis von 100 : 1 bis 10 : 1, vorzugsweise von 80 : 1 bis 20 : 1, bevorzugt von 70 : 1 bis 30 : 1, insbesondere von 55 : 1 bis 45 : 1, erhältlich ist.

Gemäß einer bevorzugten Ausführungsform der vorliegenden Erfindung weist das mindestens eine vernetzte, aminierte Siloxanpolymer b) ein mittleres Molekulargewicht $M_w$ von 2.000 bis 1.000.000 g/mol, insbesondere von 5.000 bis 200.000 g/mol, auf. Spezielle vernetzte, aminierte Siloxanpolymere, welche das zuvor angeführte mittlere Molekulargewicht $M_w$ aufweisen, resultieren in einer besonders guten Verbesserung der Farbtiefe. Das mittlere Molekulargewicht $M_w$ kann beispielsweise durch Gelpermeationschromatographie (GPC) bestimmt werden (Liu X. M. et. al.; "Comparative Studies of Poly(DimethylSiloxanes) Using Automated GPC-MALDI-TOF MS and On-Line GPC-ESI-TOF MS"; Am. Soc. Mass. Spectrom., 2003, 14, Seiten 195 bis 202).

Besonders gute Ergebnisse im Rahmen der vorliegenden Erfindung werden erhalten, wenn das mindestens eine vernetzte, aminierte Siloxanpolymer b) als Emulsion vorliegt, wobei die Emulsion eine mittlere Teilchengröße Dso von 3 bis 500 nm, vorzugsweise von 10 bis 400 nm, bevorzugt von 50 bis 300 nm, insbesondere von 100 bis 300 nm,

aufweist. Der Einsatz derartiger Emulsionen, in welcher das vernetzte, aminierte Siloxanpolymer bestimmte mittlere Teilchengrößen aufweist, führt erfindungsgemäß zu einer besonders starken Erhöhung der Farbtiefe. Die mittlere Teilchengröße $D_{50}$ kann beispielsweise durch dynamische Lichtstreuung (DLS) bestimmt werden (Bergna H. E. et. al.; "Collodial Silica-Fundamentals and Application"; Surfactant science series, CRC Press, 2006, 131, Seiten 65 bis 80).

Das mindestens eine vernetzte, aminierte Siloxanpolymer b) ist in den erfindungsgemäßen kosmetischen Mitteln in einer Gesamtmenge von 0,4 bis 0,9 Gew.-%, vorzugsweise von 0,5 bis 0,8 Gew.-%, insbesondere von 0,6 bis 0,8 Gew.-%, bezogen auf das Gesamtgewicht des kosmetischen Mittels, enthalten. Der Einsatz der zuvor genannten Gesamtmenge des speziellen vernetzten, aminierten Siloxanpolymers führt zu einer erhöhten Farbtiefe und erlaubt daher zur Erreichung einer vergleichbaren Farbtiefe gegenüber Oxidationsfärbemitteln des Standes der Technik eine signifikante Verringerung der Gesamtmenge an eingesetzten Farbstoffen. Werden hingegen Mengen von mehr als 1,0 Gew.-% des vernetzten, aminierten Siloxanpolymers eingesetzt, so wird anstelle der Erhöhung der Farbtiefe eine Verringerung der Farbtiefe beobachtet. Daher führt nur der Einsatz des vernetzten, aminierten Siloxanpolymers in den zuvor angeführten engen Mengenbereichen zu der erfindungsgemäßen Erhöhung der Farbtiefe.

Als dritten wesentlichen Bestandteil c) enthält das erfindungsgemäße kosmetische Mittel mindestens ein nichtionisches Tensid der Formel (I).

Im Rahmen der vorliegenden Erfindung ist es bevorzugt, wenn in der Formel (I) R für eine lineare oder verzweigte Alkylkette mit 12 bis 14 Kohlenstoffatomen, insbesondere mit 13 Kohlenstoffatomen, und n für ganze Zahlen von 7 bis 12, vorzugsweise von 8 bis 11, bevorzugt von 9 oder 10, insbesondere von 10, steht. Die Kombination von speziellen nichtionischen Tensiden mit vernetzten, aminierten Siloxanpolymeren in Oxidationsfärbemitteln führt zu einer Erhöhung der Farbtiefe. Besonders bevorzugt wird als nichtionisches Tensid die unter der INCI-Bezeichnung Trideceth-10 (Polyoxyethylen (10) tridecylether) bekannte Verbindung eingesetzt.

Bevorzugt wird das nichtionische Tensid in bestimmten Mengen in den erfindungsgemäßen kosmetischen Mitteln eingesetzt. Bevorzugte Ausführungsformen der vorliegenden Erfindung sind daher dadurch gekennzeichnet, dass das kosmetische Mittel das mindestens eine nichtionische Tensid c) der Formel (I) in einer Gesamtmenge von 0,0005 bis 10 Gew.-%, vorzugsweise von 0,001 bis 8,0 Gew.-%, bevorzugt von 0,005 bis 5,0 Gew.-%, insbesondere von 0,01 bis 1,0 Gew.-%, enthält. Der Einsatz von zuvor angeführten Mengen an nichtionischen Tensiden führt zu einer ausreichenden Emulgierung des teilchenförmigen vernetzten, aminierten Siloxanpolymers und gewährleistet auf diese Weise eine homogene Verteilung des vernetzten, aminierten Siloxanpolymers in den erfindungsgemäßen kosmetischen Mitteln. Hierdurch wird eine homogene Färbung ermöglicht und die gleichmäßige Erhöhung der Farbtiefe an allen Stellen der keratinischen Fasern sichergestellt.

Es hat sich gezeigt, dass ein Zusatz an speziellen Dimethylcyclosiloxanen das mindestens eine vernetzte, aminierte Siloxanpolymer in den erfindungsgemäßen kosmetischen Mitteln stabilisieren kann, so dass die Erhöhung der Farbtiefe verstärkt wird. Erfindungsgemäß bevorzugte kosmetische Mittel enthalten daher zusätzlich mindestens ein Dimethylcyclosiloxan in einer Gesamtmenge von 0,001 bis 2,0 Gew.-%, vorzugsweise von 0,05 bis 1,5 Gew.-%, insbesondere von 0,01 bis 1,0 Gew.-%, bezogen auf das Gesamtgewicht des kosmetischen Mittels, enthält, wobei das mindestens eine Dimethylcyclosiloxan die Formel (II) aufweist

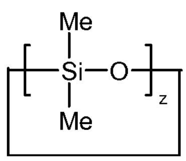

(II),

worin

z für ganze Zahlen von 2 bis 8, vorzugsweise von 2 bis 6, bevorzugt von 2 bis 4, insbesondere für die ganze Zahl 4, steht.

Die erfindungsgemäßen kosmetischen Mittel können weitere Wirk- und Zusatzstoff enthalten. Es ist daher im Rahmen der vorliegenden Erfindung bevorzugt, wenn das kosmetische Mittel zusätzlich mindestens eine weitere Verbindung, ausgewählt aus der Gruppe von (i) Verdickungsmitteln; (ii) linearen oder verzweigten, gesättigten oder ungesättigten Alkoholen mit 8 bis 20 Kohlenstoffatomen; (iii) Tensiden, insbesondere amphoteren Tensiden; (iv) Alkalisierungsmitteln; (v) Ölen; sowie (vi) deren Mischungen, enthält.

Bevorzugt werden die erfindungsgemäßen kosmetischen Mittel als fließfähige Zubereitungen formuliert. Dabei sollten die kosmetischen Mittel so formuliert werden, dass diese sich einerseits gut am Anwendungsort auftragen und verteilen lassen, andererseits jedoch ausreichend viskos sind, so dass sie während der Einwirkzeit am Wirkort verbleiben und nicht verlaufen.

Es hat sich daher erfindungsgemäß als vorteilhaft erwiesen, wenn die erfindungsgemäßen kosmetischen Mittel mindestens ein Verdickungsmittel aus der Gruppe von (i) anionischen, synthetischen Polymeren; (ii) kationischen, synthetischen Polymeren; (iii) natürlich vorkommenden Verdickungsmitteln, wie nichtionischen Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektinen, Alginaten, Stärke-Fraktionen und Derivaten, wie Amylose, Amylopektin und Dextrinen, sowie Cellulosederivaten, wie beispielsweise Methylcellulose, Carboxyalkylcellulosen und Hydroxyalkylcellulosen; (iv) nichtionischen, synthetischen Polymeren, wie Polyvinylalkohol oder Polyvinylpyrrolidinon; (v) anorganischen Verdickungsmitteln, insbesondere Schichtsilikaten wie beispielsweise Bentonit, besonders Smektite, wie Montmorillonit oder Hectorit; sowie (vi) deren Mischungen, in einer Gesamtmenge von 0,0005 bis 5,0 Gew.-%, vorzugsweise von 0,001 bis 3,0 Gew.-%, bevorzugt von 0,005 bis 1,0 Gew.-%, insbesondere von 0,008 bis 0,01 Gew.-%, bezogen auf das Gesamtgewicht des kosmetischen Mittels, enthalten.

Es hat sich in diesem Zusammenhang als vorteilhaft erwiesen, wenn als Verdickungsmittel mindestens ein natürlich vorkommendes Verdickungsmittel, insbesondere Xanthangum sowie dessen Salze, in einer Gesamtmenge von 0,0005 bis 5,0 Gew.-%, vorzugsweise von 0,001 bis 1,0 Gew.-%, bevorzugt von 0,005 bis 0,5 Gew.-%, insbesondere von 0,01 bis 0,2 Gew.-%, bezogen auf das Gesamtgewicht des kosmetischen Mittels, enthalten ist.

Weiterhin können die erfindungsgemäßen kosmetischen Mittel mindestens einen linearen oder verzweigten, gesättigten oder ungesättigten Alkohol mit 8 bis 20 Kohlenstoffatomen enthalten. In diesem Zusammenhang kann es bevorzugt sein, wenn der lineare oder verzweigte, gesättigte oder ungesättigte Alkohol mit 8 bis 20 Kohlenstoffatomen ausgewählt ist aus der Gruppe von Myristylalkohol (1-Tetradecanol), Stearylalkohol (1-Octadecanol), Cetearylalkohol, 2-Octyldodecanol, Arachylalkohol (Eicosan-1-ol), Gadoleylalkohol ((9Z)-Eicos-9-en-1-ol), Arachidonalkohol ((5Z,8Z,11Z,14Z)-Eicosa-5,8,11,14-tetraen-1-ol), vorzugsweise 2-Octyldodecanol und/oder Cetearylalkohol, und in einer Gesamtmenge von 1,0 bis 35 Gew.-%, vorzugsweise von 5,0 bis 30 Gew.-%, bevorzugt von 10 bis 25 Gew.-%, insbesondere von 12 bis 20 Gew.-%, bezogen auf das Gesamtgewicht des kosmetischen Mittels, enthalten ist.

Bevorzugt können die erfindungsgemäßen kosmetischen Mittel weiterhin mindestens einen Partialester aus einem Polyol mit 2 bis 6 Kohlenstoffatomen und linearen gesättigten Carbonsäuren mit 12 bis 30, insbesondere 14 bis 22 Kohlenstoffatomen, wobei die Partialester hydroxyliert sein können, in einer Gesamtmenge von 0,5 bis 10 Gew.-%, insbesondere von 3,0 bis 8,0 Gew.-%, bezogen auf das Gesamtgewicht des kosmetischen Mittels, enthalten. Solche Partialester sind insbesondere die Mono- und Diester von Glycerin oder die Monoester von Propylenglycol oder die Mono- und Diester von Ethylenglycol oder die Mono-, Di-, Tri- und Tetraester von Pentaerythrit jeweils mit linearen gesättigten $C_{12}$ - $C_{30}$-Carbonsäuren, welche hydroxyliert sein können, insbesondere diejenigen mit Palmitin- und Stearinsäure, die Sorbitanmono-, -di- oder -triester von linearen gesättigten $C_{12}$ - $C_{30}$-Carbonsäuren, welche hydroxyliert sein können, insbesondere diejenigen von Myristinsäure, Palmitinsäure, Stearinsäure oder von Mischungen dieser Fettsäuren und die Methylglucosemono- und -diester von linearen gesättigten $C_{12}$ - $C_{30}$-Carbonsäuren, welche hydroxyliert sein können.

In diesem Zusammenhang kann es daher vorgesehen sein, dass die erfindungsgemäßen kosmetischen Mittel mindestens einen Polyolpartialester, ausgewählt aus Glycerinmonostearat, Glycerinmonopalmitat, Glycerindistearat, Glycerindipalmitat, Ethylenglycolmonostearat, Ethylenglycolmonopalmitat, Ethylenglycoldistearat, Ethylenglycoldipalmitat, sowie Mischungen hiervon, insbesondere Mischungen aus Glycerinmonostearat, Glycerinmonopalmitat, Glycerindistearat und Glycerindipalmitat in einer Gesamtmenge von 0,5 bis 10 Gew.-%, insbesondere von 3,0 bis 8,0 Gew.-%, bezogen auf das Gesamtgewicht des kosmetischen Mittels, enthalten.

Der Einsatz der zuvor angeführten Alkohole, Partialester und Polypartialester in den erfindungsgemäßen kosmetischen Mitteln kann insbesondere dann bevorzugt sein, wenn die erfindungsgemäßen kosmetischen Mittel in Form einer Öl-in-Wasser-Emulsion vorliegen.

Weiterhin kann es erfindungsgemäß vorgesehen sein, dass die kosmetischen Mittel gemäß der Erfindung mindestens ein Tensid enthalten, welches von dem nichtionischen Tensid c) der Formel (I) verschieden ist.

Gemäß einer bevorzugten Ausführungsform der vorliegenden Erfindung enthalten die erfindungsgemäßen kosmetischen Mittel mindestens ein amphoteres Tensid in einer Gesamtmenge von 0,1 bis 5,0 Gew.-%, insbesondere von 0,2 bis 2,0 Gew.-%, bezogen auf das Gesamtgewicht des kosmetischen Mittels. Als amphotere bzw. zwitterionische Tenside werden solche oberflächenaktiven Verbindungen bezeichnet, welche mindestens eine quartäre Ammoniumgruppe und mindestens eine - $COO^{(-)}$- oder -$SO3^{(-)}$-Gruppe aufweisen.

Als amphotere Tenside sind im Rahmen der vorliegenden Erfindung die nachfolgend genannten Verbindungen besonders bevorzugt:

- Alkylbetaine mit 8 bis 20 Kohlenstoffatomen in der Alkylgruppe,
- Amidopropylbetaine mit 8 bis 20 Kohlenstoffatomen in der Acylgruppe,
- Sulfobetaine mit 8 bis 20 Kohlenstoffatomen in der Acylgruppe, und
- Amphoacetate oder Amphodiacetate mit 8 bis 20 Kohlenstoffatomen in der Acylgruppe.

In einer besonders bevorzugten Ausführungsform enthalten die erfindungsgemäßen kosmetischen Mittel als Tensid mindestens ein amphoteres Tensid, ausgewählt aus Amidopropylbetainen mit 9 bis 13 Kohlenstoffatomen in der Acylgruppe, in einer Gesamtmenge von 0,1 bis 5,0 Gew.-%, insbesondere von 0,2 bis 2,0 Gew.-%, bezogen auf das

Gesamtgewicht des kosmetischen Mittels.

Weiterhin kann es vorgesehen sein, dass die erfindungsgemäßen kosmetischen Mittel mindestens ein ethoxyliertes nichtionisches Tensid, welches von dem nichtionischen Tensid c) der Formel (I) verschieden ist, in einer Gesamtmenge von 0,5 bis 6,0 Gew.-%, insbesondere von 1,0 bis 4,0 Gew.-%, bezogen auf das Gesamtgewicht des kosmetischen Mittels, enthalten. Dabei hat es sich als besonders vorteilhaft erwiesen, wenn das ethoxylierte nichtionische Tensid einen HLB-Wert oberhalb von 10, vorzugsweise oberhalb von 13 aufweist. Hierzu ist es erforderlich, dass das nichtionische Tensid einen ausreichend hohen Ethoxylierungsgrad besitzt. In diesem Zusammenhang enthält das erfindungsgemäße kosmetische Mittel daher als ethoxyliertes nichtionisches Tensid mindestens ein ethoxyliertes Tensid mit mindestens 12 Ethylenoxideinheiten. Neben den entsprechend ethoxylierten Fettalkoholen, insbesondere Laurylalkohol, Myristylalkohol, Cetylalkohol, Stearylalkohol, Arachylalkohol und Behenylalkohol, sind erfindungsgemäß insbesondere die Anlagerungsprodukte von 20 bis 60 Mol Ethylenoxid an Rizinusöl und gehärtetes Rizinusöl besonders geeignet. Das mindestens eine ethoxylierte nichtionische Tensid ist bevorzugt ausgewählt aus Tensiden mit der INCI-Bezeichnung Ceteth-12, Steareth-12, Ceteareth-12, Ceteth-20, Steareth-20, Ceteareth-20, Ceteth-30, Steareth-30, Ceteareth-30, Oleth-30, Ceteareth-50, PEG-40 Hydrogenated Castor Oil und PEG-60 Hydrogenated Castor Oil sowie Mischungen dieser Substanzen, besonders bevorzugt ausgewählt aus Ceteth-20, Steareth-20, Ceteareth-20, Ceteth-30, Steareth-30 und Ceteareth-30.

Kosmetische Mittel im Rahmen der vorliegenden Erfindung weisen in der Regel einen basischen pH-Wert, insbesondere zwischen pH 8,0 und pH 12, auf. Diese pH-Werte sind erforderlich, um eine Öffnung der äußeren Schuppenschicht (Cuticula) zu gewährleisten und eine Penetration der Oxidationsfarbstoffvorprodukte und/oder des Oxidationsmittels in das Haar zu ermöglichen.

Die Einstellung des zuvor genannten pH-Wertes kann bevorzugt unter Verwendung eines Alkalisierungsmittels erfolgen. Im Rahmen der vorliegenden Erfindung ist das Alkalisierungsmittel ausgewählt aus der Gruppe von (i) anorganischen Alkalisierungsmitteln; (ii) organischen Alkalisierungsmitteln; sowie (iii) deren Mischungen, und in einer Gesamtmenge von 1,5 bis 9,5 Gew.-%, vorzugsweise von 2,5 bis 8,5 Gew.-%, bevorzugt von 3,0 bis 8,0 Gew.-%, insbesondere von 3,5 bis 7,5 Gew.-%, bezogen auf das Gesamtgewicht des kosmetischen Mittels, enthalten.

Bevorzugte anorganische Alkalisierungsmittel sind ausgewählt aus der Gruppe, welche gebildet wird aus Ammoniak bzw. Ammoniumhydroxid, also wässrigen Lösungen von Ammoniak, Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat sowie Mischungen hiervon. Ammoniak bzw. Ammoniumhydroxid ist ein besonders bevorzugtes Alkalisierungsmittel. Besonders bevorzugt ist Ammoniak in einer Gesamtmenge von 0,1 bis 20 Gew.-%, vorzugsweise von 0,5 bis 10 Gew.-%, insbesondere von 1,0 bis 7,0 Gew.-%, bezogen auf das Gesamtgewicht des kosmetischen Mittels, enthalten.

Bevorzugte organische Alkalisierungsmittel sind ausgewählt aus mindestens einem Alkanolamin. Erfindungsgemäß bevorzugte Alkanolamine sind ausgewählt aus Alkanolaminen aus primären, sekundären oder tertiären Aminen mit einem $C_2$-$C_6$-Alkylgrundkörper, welcher mindestens eine Hydroxylgruppe trägt. Besonders bevorzugte Alkanolamine werden aus der Gruppe ausgewählt, welche gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methylpropan-2-ol, 3-Aminopropan-1,2-diol, 2-Amino-2-methylpropan-1,3-diol, 2-Amino-2-ethyl-1,3-propandiol, N,N-Dimethyl-ethanolamin, Triethanolamin, Diethanolamin und Triisopropanolamin. Erfindungsgemäß ganz besonders bevorzugte Alkanolamine werden ausgewählt aus der Gruppe von 2-Aminoethan-1-ol (Monoethanolamin), 2-Amino-2-methyl-propan-1-ol, 2-Amino-2-methyl-propan-1,3-diol und Triethanolamin. Besonders bevorzugte erfindungsgemäße kosmetische Mittel enthalten eine Mischung aus Monoethanolamin und 2-Amino-2-methylpropan-1ol. Bevorzugt ist das mindestens eine Alkanolamin in einer Gesamtmenge von 0,05 bis 15 Gew.-%, vorzugsweise von 0,5 bis 10 Gew.-%, insbesondere von 3,5 bis 7,5 Gew.-%, bezogen auf das Gesamtgewicht des kosmetischen Mittels, enthalten.

Weitere erfindungsgemäß bevorzugte organische Alkalisierungsmittel sind ausgewählt aus basischen Aminosäuren, besonders bevorzugt ausgewählt aus der Gruppe, welche gebildet wird aus L-Arginin, D-Arginin, D/L-Arginin, L-Lysin, D-Lysin, D/L-Lysin sowie Mischungen hiervon. Erfindungsgemäß besonders bevorzugte basische Aminosäuren sind ausgewählt aus L-Arginin, D-Arginin und D/L-Arginin. Bevorzugte erfindungsgemäße kosmetische Mittel enthalten mindestens ein von Alkanolaminen und Ammoniak verschiedenes Alkalisierungsmittel in einer Gesamtmenge von 0,05 bis 5,0 Gew.-%, insbesondere von 0,5 bis 3,0 Gew.-%, bezogen auf das Gesamtgewicht des kosmetischen Mittels.

In einer besonders bevorzugten Ausführungsform enthalten die erfindungsgemäßen kosmetischen Mittel als Alkalisierungsmittel eine Mischung aus mindestens zwei voneinander verschiedenen Alkanolaminen, insbesondere von Monoethanolamin und 2-Amino-2-methylpropan-1-ol, in einer Gesamtmenge von 0,05 bis 15 Gew.-%, vorzugsweise von 0,5 bis 10 Gew.-%, insbesondere von 3,5 bis 7,5 Gew.-%, bezogen auf das Gesamtgewicht des kosmetischen Mittels.

Bevorzugt beträgt der pH-Wert der erfindungsgemäßen kosmetischen Mittel, gemessen bei 22°C, 8 bis 13, vorzugsweise 9,5 bis 12, bevorzugt 10 bis 11,5, insbesondere 10,5 bis 11.

Im Rahmen der vorliegenden Erfindung kann es weiterhin bevorzugt sein, wenn die erfindungsgemäßen kosmetischen Mittel mindestens ein Öl, ausgewählt aus der Gruppe von Sonnenblumenöl, Maisöl, Sojaöl, Kürbiskernöl, Traubenkernöl, Sesamöl, Haselnussöl, Aprikosenkernöl, Macadamianussöl, Araraöl, Rizinusöl, Avocadoöl sowie deren Mischungen, in einer Gesamtmenge von 0,1 bis 10 Gew.-%, bevorzugt von 0,2 bis 5,0 Gew.-%, insbesondere von 0,5 bis 2,0 Gew.-%, bezogen auf das Gesamtgewicht des kosmetischen Mittels, enthalten.

Besonders bevorzugt enthalten die erfindungsgemäßen kosmetischen Mittel Traubenkernöl in einer Gesamtmenge von 0,1 bis 10 Gew.-%, bevorzugt von 0,2 bis 5,0 Gew.-%, insbesondere von 0,5 bis 2,0 Gew.-%, bezogen auf das Gesamtgewicht des kosmetischen Mittels.

Gemäß einer besonders bevorzugten Ausführungsform der vorliegenden Erfindung enthalten die als Öl-in-Wasser-Emulsion vorliegenden erfindungsgemäßen kosmetischen Mittel - bezogen auf das Gesamtgewicht der kosmetischen Mittel -

- Cetearylalkohol in einer Gesamtmenge von 2,0 bis 20 Gew.-%, insbesondere von 5,0 bis 18 Gew.-%, weiterhin
- Mischungen aus Glycerinmonostearat, Glycerinmonopalmitat, Glycerindistearat und Glycerindipalmitat in einer Gesamtmenge von 0,5 bis 10 Gew.-%, bevorzugt 3,0 bis 8,0 Gew.-%, weiterhin
- mindestens ein amphoteres Tensid, ausgewählt aus Amidopropylbetainen mit 9 bis 13 Kohlenstoffatomen in der Acylgruppe, in einer Gesamtmenge von 0,1 bis 5,0 Gew.-%, insbesondere von 0,2 bis 2,0 Gew.-%, weiterhin
- eine Mischung aus mindestens zwei voneinander verschiedenen Alkanolaminen, insbesondere von Monoethanolamin und 2-Amino-2-methylpropan-1-ol, in einer Gesamtmenge von 0,05 bis 15 Gew.-%, vorzugsweise von 0,5 bis 10 Gew.-%, insbesondere von 3,5 bis 7,5 Gew.-%, weiterhin
- Traubenkernöl in einer Gesamtmenge von 0,1 bis 10 Gew.-%, bevorzugt von 0,2 bis 5,0 Gew.-%, insbesondere von 0,5 bis 2,0 Gew.-%.

Oxidative Färbezusammensetzungen können auch direkt vor der Anwendung aus zwei oder mehreren getrennt verpackten Zusammensetzungen hergestellt werden. Dies bietet sich insbesondere zur Trennung inkompatibler Inhaltsstoffe an, um eine vorzeitige Reaktion zu vermeiden. Eine Auftrennung in Mehrkomponentensysteme ist insbesondere dort bevorzugt, wo Inkompatibilitäten der Inhaltsstoffe zu erwarten oder zu befürchten sind. Die oxidative Färbezusammensetzung wird in diesen Fällen vom Verbraucher direkt vor der Anwendung durch Vermischen der Komponenten hergestellt. Im Rahmen der vorliegenden Erfindung ist dieses Vorgehen bei oxidativen Färbemitteln, bei welchen das erfindungsgemäße kosmetische Mittel zunächst getrennt von einer Oxidationsmittelzubereitung, enthaltend mindestens ein Oxidationsmittel, vorliegt, besonders bevorzugt.

Ein weiterer Gegenstand der vorliegenden Erfindung ist daher eine Verpackungseinheit (Kit-of-Parts), umfassend - getrennt voneinander konfektioniert -

a) mindestens einen Container (C1), enthaltend ein erfindungsgemäßes kosmetisches Mittel, und

b) mindestens einen Container (C2), enthaltend eine Oxidationsmittelzubereitung, welche in einem kosmetisch verträglichen Träger mindestens ein Oxidationsmittel sowie mindestens eine Säure enthält.

Unter dem Begriff "Container" wird im Rahmen der vorliegenden Erfindung eine Umhüllung verstanden, welche in Form einer gegebenenfalls wieder verschließbaren Flasche, einer Tube, einer Dose, eines Tütchens, eines Sachets oder ähnlichen Umhüllungen vorliegt. Dem Umhüllungsmaterial sind erfindungsgemäß keine Grenzen gesetzt. Bevorzugt handelt es sich dabei jedoch um Umhüllungen aus Glas oder Kunststoff.

Die Oxidationsmittel im Rahmen der vorliegenden Erfindung sind von Luftsauerstoff verschieden. Als Oxidationsmittel können Wasserstoffperoxid sowie dessen feste Anlagerungsprodukte an organische und anorganische Verbindungen eingesetzt werden. Als feste Anlagerungsprodukte kommen erfindungsgemäß insbesondere die Anlagerungsprodukte an Harnstoff, Melamin, Polyvinylpyrrolidinon sowie Natriumborat in Frage. Wasserstoffperoxid und/oder eines seiner festen Anlagerungsprodukte an organische oder anorganische Verbindungen sind als Oxidationsmittel besonders bevorzugt. Erfindungsgemäß bevorzugt ist das Oxidationsmittel daher ausgewählt aus der Gruppe von Persulfaten, Chloriten, Wasserstoffperoxid und Anlagerungsprodukten von Wasserstoffperoxid an Harnstoff, Melamin sowie Natriumborat, insbesondere Wasserstoffperoxid.

Ein besonders bevorzugte Ausführungsform der vorliegenden Erfindung ist somit dadurch gekennzeichnet, dass das Oxidationsmittel in der Oxidationsmittelzubereitung (M2) Wasserstoffperoxid ist und in einer Gesamtmenge von 0,5 bis 7,0 Gew.-%, vorzugsweise von 1,0 bis 7,0 Gew.-%, insbesondere von 3,0 bis 7,0 Gew.-%, bezogen auf das Gesamtgewicht der Oxidationsmittelzubereitung (M2), enthalten ist. Die Berechnung der Gesamtmenge bezieht sich hierbei auf 100 %-iges $H_2O_2$.

Die Oxidationsmittelzubereitungen können weiterhin Wasser in einer Gesamtmenge von 40 bis 98 Gew.-%, insbesondere von 65 bis 85 Gew.-%, bezogen auf das Gesamtgewicht der Oxidationsmittelzubereitung (M2), enthalten.

Gemäß einer bevorzugten Ausführungsform der vorliegenden Erfindung enthalten die Oxidationsmittelzubereitungen weiterhin mindestens ein lineares gesättigtes Alkanol mit 12 bis 30 Kohlenstoffatomen, insbesondere mit 16 bis 22 Kohlenstoffatomen, in einer Gesamtmenge von 0,1 bis 10 Gew.-%, vorzugsweise von 0,5 bis 5,0 Gew.-%, insbesondere von 1,0 bis 4,0 Gew.-%, bezogen auf das Gesamtgewicht der Oxidationsmittelzubereitung (M2). Bevorzugt sind insbesondere Cetylalkohol, Stearylalkohol, Arachidylalkohol, Behenylalkohol und Lanolinalkohol oder Gemische dieser Alkohole, wie sie bei der großtechnischen Hydrierung von pflanzlichen und tierischen Fettsäuren erhältlich sind, sowie Mischungen dieser Alkanole. Besonders bevorzugt ist die Mischung Cetearylalkohol.

In einer weiteren bevorzugten Ausführungsform der vorliegenden Erfindung enthalten die Oxidationsmittelzubereitungen mindestens ein ethoxyliertes nichtionisches Tensid, welches bevorzugt ausgewählt ist aus Tensiden mit der INCI-Bezeichnung Ceteth-12, Steareth-12, Ceteareth-12, Ceteth-20, Steareth-20, Ceteareth-20, Ceteth-30, Steareth-30, Ceteareth-30, Oleth-30, Ceteareth-50, PEG-40 Hydrogenated Castor Oil und PEG-60 Hydrogenated Castor Oil sowie Mischungen dieser Substanzen, besonders bevorzugt ausgewählt aus Ceteth-20, Steareth-20, Ceteareth-20, Ceteth-30, Steareth-30 und Ceteareth-30, in einer Gesamtmenge von 0,1 bis 10 Gew.-%, vorzugsweise von 0,5 bis 5,0 Gew.-%, insbesondere von 1 bis 4,0 Gew.-%, bezogen auf das Gesamtgewicht der Oxidationsmittelzubereitung (M2).

Im Rahmen der vorliegenden Erfindung kann es darüber hinaus auch vorgesehen sein, dass die Oxidationsmittelzubereitungen mindestens einen Ester aus einer Carbonsäure mit 10 bis 20 Kohlenstoffatomen und einem linearen oder verzweigten Alkohol mit 1 bis 5 Kohlenstoffatomen, insbesondere Isopropylmyristat, in einer Gesamtmenge von 3,0 bis 25 Gew.-%, vorzugsweise von 5,0 bis 20 Gew.-%, insbesondere von 8,0 bis 15 Gew.-%, bezogen auf das Gesamtgewicht der Oxidationsmittelzubereitung (M2), enthalten.

Gemäß einer besonders bevorzugten Ausführungsform der vorliegenden Erfindung enthalten die Oxidationsmittelzubereitungen (M2) - bezogen auf das Gesamtgewicht der Oxidationsmittelzubereitungen (M2) -

- mindestens ein lineares gesättigtes Alkanol mit 12 bis 30 Kohlenstoffatomen in einer Gesamtmenge von 0,1 bis 10 Gew.-%, vorzugsweise von 0,5 bis 5,0 Gew.-%, insbesondere von 1,0 bis 4,0 Gew.-%, weiterhin
- mindestens ein ethoxyliertes nichtionisches Tensid, welches bevorzugt ausgewählt ist aus Tensiden mit der INCI-Bezeichnung Ceteth-12, Steareth-12, Ceteareth-12, Ceteth-20, Steareth-20, Ceteareth-20, Ceteth-30, Steareth-30, Ceteareth-30, Oleth-30, Ceteareth-50, PEG-40 Hydrogenated Castor Oil und PEG-60 Hydrogenated Castor Oil sowie Mischungen dieser Substanzen, besonders bevorzugt ausgewählt aus Ceteth-20, Steareth-20, Ceteareth-20, Ceteth-30, Steareth-30 und Ceteareth-30, in einer Gesamtmenge von 0,1 bis 10 Gew.-%, vorzugsweise von 0,5 bis 5,0 Gew.-%, insbesondere von 1,0 bis 4,0 Gew.-%, sowie
- mindestens einen Ester aus einer Carbonsäure mit 10 bis 20 Kohlenstoffatomen und einem linearen oder verzweigten Alkohol mit 1 bis 5 Kohlenstoffatomen, bevorzugt Isopropylmyristat, in einer Gesamtmenge von 3,0 bis 25 Gew.-%, vorzugsweise von 5,0 bis 20 Gew.-%, insbesondere von 8,0 bis 15 Gew.-%.

Die erfindungsgemäßen Oxidationsmittelzubereitungen enthalten weiterhin mindestens eine Säure. Bevorzugte Säuren sind ausgewählt aus Dipicolinsäure, Genusssäuren, wie beispielsweise Zitronensäure, Essigsäure, Apfelsäure, Milchsäure und Weinsäure, verdünnten Mineralsäuren, wie Salzsäure, Phosphorsäure, Pyrophosphorsäure und Schwefelsäure, sowie Mischungen hiervon.

Die Oxidationsmittelzubereitungen weisen bevorzugt einen pH-Wert, gemessen bei 22 °C, im Bereich von 2 bis 5, insbesondere von 3 bis 4, auf.

Zur Herstellung von oxidativen Färbezusammensetzungen aus der erfindungsgemäßen Verpackungseinheit (Kit-of-parts) wird das erfindungsgemäße kosmetische Mittel (M1) in dem Container (C1) mit der Oxidationsmittelzubereitung (M2) in dem Container (C2) oder *vice versa* vermischt.

Weiterhin kann es erfindungsgemäß besonders vorteilhaft sein, wenn die Verpackungseinheit mindestens ein weiteres Haarbehandlungsmittel in einem zusätzlichen Container enthält, insbesondere eine Konditioniermittelzubereitung. Diese Konditioniermittelzubereitung enthält vorteilhafterweise mindestens ein Konditioniermittel, ausgewählt aus der Gruppe von kationischen Polymeren, Siliconderivaten und Ölen. Darüber hinaus kann die Verpackungseinheit Applikationshilfen, wie Kämme, Bürsten, Applicetten oder Pinsel, persönliche Schutzkleidung, insbesondere Ein-Weg-Handschuhe, sowie gegebenenfalls eine Gebrauchsanleitung umfassen. Unter einer Applicette wird ein breiter Pinsel verstanden, an dessen Stielende sich eine Spitze befindet, welche das Abteilen von Faserbündeln bzw. Strähnchen aus der Gesamtmenge der Fasern erlaubt und vereinfacht.

Bezüglich des erfindungsgemäßen kosmetischen Mittels (M1) in dem Container (C1) und der Oxidationsmittelzubereitung (M2) in dem Container (C2) gilt mutatis mutandis das zu den erfindungsgemäßen kosmetischen Mitteln Gesagte.

Ein weiterer Gegenstand der vorliegenden Erfindung ist ein Verfahren zur Färbung keratinischer Fasern, wobei das Verfahren die folgenden Verfahrensschritte umfasst:

a) Bereitstellen eines erfindungsgemäßen kosmetischen Mittels (M1),
b) Bereitstellen einer Oxidationsmittelzubereitung (M2), enthaltend in einem kosmetisch verträglichen Träger mindestens ein Oxidationsmittel sowie mindestens eine Säure,

c) Vermischen des kosmetischen Mittels (M1) mit der Oxidationsmittelzubereitung (M2),

d) Applizieren der in Schritt c) erhaltenen Mischung auf die keratinischen Fasern und Belassen dieser Mischung für eine Zeit von 10 bis 60 Minuten, bevorzugt von 20 bis 45 Minuten, bei Raumtemperatur und/oder bei mindestens 30 °C auf den keratinischen Fasern,

e) Spülen der keratinischen Fasern mit Wasser und/oder einer reinigenden Zusammensetzung für 1 bis 5 Minuten, und

f) gegebenenfalls Applizieren eines Nachbehandlungsmittels auf die keratinischen Fasern und Ausspülen nach einer Zeit von 1 bis 10 Minuten.

Das erfindungsgemäße Verfahren zur Färbung keratinischer Fasern unter Verwendung einer Kombination aus einem speziellen vernetzten, aminierten Siloxanpolymer und einem nichtionischen Tensid resultiert in einer erhöhten Farbtiefe der gefärbten keratinischen Fasern. Hierdurch kann gegenüber Färbemitteln des Standes der Technik zur Erreichung einer vergleichbaren Farbtiefe die Gesamtmenge an Farbstoffen signifikant verringert werden.

Unter Raumtemperatur ist dabei im Rahmen der vorliegenden Erfindung die Umgebungstemperatur zu verstehen. Die Wirkung der Färbe- und/oder Aufhellzubereitung kann durch externe Wärmezufuhr, beispielsweise mittels einer Wärmehaube, verstärkt werden. Die bevorzugte Einwirkdauer der Färbe- und/oder Aufhellzubereitung auf die keratinische Faser beträgt 10 bis 60 min, bevorzugt 20 bis 45 min. Nach Beendigung der Einwirkdauer wird das verbliebene Färbemittel aus den keratinischen Fasern mit Hilfe einer Reinigungszubereitung, welche bevorzugt mindestens ein kationisches und/oder anionisches und/oder nichtionisches Tensid enthält, und/oder Wasser ausgewaschen. Gegebenenfalls wird der Vorgang mit einem weiteren Mittel wiederholt. Nach dem Auswaschen werden die keratinischen Fasern gegebenenfalls mit einem Nachbehandlungsmittel, beispielsweise einem Konditioniermittel, gespült und mit einem Handtuch oder einem Heißluftgebläse getrocknet. Die Auftragung der Färbezubereitung erfolgt üblicherweise mit der Hand durch den Anwender. Bevorzugt wird dabei persönliche Schutzkleidung getragen, insbesondere geeignete Schutzhandschuhe, beispielsweise aus Kunststoff oder Latex zur einmaligen Benutzung (Einweghandschuhe) sowie gegebenenfalls eine Schürze. Es ist aber auch möglich, die Färbemittel mit einer Applikationshilfe auf die keratinischen Fasern aufzutragen.

Bezüglich des erfindungsgemäßen kosmetischen Mittels (M1), der Oxidationsmittel-zubereitung (M2) sowie weiterer bevorzugter Ausführungsformen des erfindungsgemäßen Verfahrens gilt mutatis mutandis das zu den erfindungsgemäßen kosmetischen Mitteln sowie zu der erfindungsgemäßen Verpackungseinheit Gesagte.

Zudem ist ein weiterer Gegenstand der vorliegenden Erfindung die Verwendung eines erfindungsgemäßen kosmetischen Mittels zur Erhöhung der Farbtiefe. Der Einsatz der Kombination aus einem speziellen vernetzten, aminierten Siloxanpolymer mit einem nichtionischen Tensid führt zu einer erhöhten Farbtiefe und erlaubt daher die signifikante Reduktion der Gesamtmenge an Farbstoffen zur Erreichung einer vergleichbaren Farbtiefe gegenüber Färbemitteln des Standes der Technik.

Bezüglich weiterer bevorzugter Ausführungsformen der erfindungsgemäßen Verwendung gilt mutatis mutandis das zu den erfindungsgemäßen kosmetischen Mitteln, zu der erfindungsgemäßen Verpackungseinheit sowie zu dem erfindungsgemäßen Verfahren Gesagte.

Schließlich ist ein weiterer Gegenstand der vorliegenden Erfindung die Verwendung einer erfindungsgemäßen Verpackungseinheit (Kit-of-parts) zur Herstellung eines kosmetischen Mittels zur Farbveränderung keratinischer Fasern mit erhöhter Farbtiefe.

Bezüglich weiterer bevorzugter Ausführungsformen der erfindungsgemäßen Verwendung gilt mutatis mutandis das zu den erfindungsgemäßen kosmetischen Mitteln, zu der erfindungsgemäßen Verpackungseinheit sowie zu dem erfindungsgemäßen Verfahren Gesagte.

Die vorliegende Erfindung wird insbesondere durch nachfolgende Punkte skizziert:

1. Kosmetisches Mittel zur Farbveränderung keratinischer Fasern, enthaltend in einem kosmetisch verträglichen Träger

a) mindestens eine Verbindung, ausgewählt aus der Gruppe von Oxidationsfarbstoffvorprodukten, direktziehenden Farbstoffen sowie deren Mischungen,

b) mindestens ein vernetztes, aminiertes Siloxanpolymer, welches durch Umsetzung eines Copolymers, bestehend aus 3-(2-Aminoethylamino)propylmethylsiloxyeinheiten und Dimethylsiloxyeinheiten, mit N-Morpholinomethyltriethoxysilan erhältlich ist, in einer Gesamtmenge von 0,3 bis 1,0 Gew.-%, bezogen auf das Gesamtgewicht des kosmetischen Mittels, und

c) mindestens ein nichtionisches Tensid der Formel (I)

$$R\text{-}O\text{-}(CH_2\text{-}CH_2\text{-}O)_n\text{-}H \qquad (I),$$

worin

R für eine lineare oder verzweigte Alkylkette mit 12 bis 16 Kohlenstoffatomen steht, und
n für ganze Zahlen von 6 bis 15 steht.

2. Kosmetisches Mittel nach Punkt 1, dadurch gekennzeichnet, dass das kosmetische Mittel das Oxidationsfarbstoffvorprodukt, insbesondere die Entwickler- und/oder Kupplerkomponente(n), in einer Gesamtmenge von 0,001 bis 5,0 Gew.-%, vorzugsweise von 0,01 bis 4,0 Gew.-%, bevorzugt von 0,1 bis 3,0 Gew.-%, insbesondere von 0,5 bis 2,0 Gew.-%, bezogen auf das Gesamtgewicht des kosmetischen Mittels, enthält.
3. Kosmetisches Mittel nach einem der Punkte 1 oder 2, dadurch gekennzeichnet, dass das mindestens eine vernetzte, aminierte Siloxanpolymer b) durch die Umsetzung eines Copolymers, bestehend aus 3-(2-Aminoethylamino)propylmethylsiloxyeinheiten und Dimethylsiloxyeinheiten, mit N-Morpholinomethyltriethoxysilan in einem Gewichtsverhältnis von 100 : 1 bis 10 : 1, vorzugsweise von 80 : 1 bis 20 : 1, bevorzugt von 70 : 1 bis 30 : 1, insbesondere von 55 : 1 bis 45 : 1, erhältlich ist.
4. Kosmetisches Mittel nach einem der vorangehenden Punkte, dadurch gekennzeichnet, dass das mindestens eine vernetzte, aminierte Siloxanpolymer b) ein mittleres Molekulargewicht $M_w$ von 2.000 bis 1.000.000 g/mol, insbesondere von 5.000 bis 200.000 g/mol, aufweist.
5. Kosmetisches Mittel nach einem der vorangehenden Punkte, dadurch gekennzeichnet, dass das mindestens eine aminierte Siloxanpolymer b) als Emulsion vorliegt, wobei die Emulsion eine mittlere Teilchengröße Dso von 3 bis 500 nm, vorzugsweise von 10 bis 400 nm, bevorzugt von 50 bis 300 nm, insbesondere von 100 bis 300 nm, aufweist.
6. Kosmetisches Mittel nach einem der vorangehenden Punkte, dadurch gekennzeichnet, dass das kosmetische Mittel das mindestens eine vernetzte, aminierte Siloxanpolymer b) in einer Gesamtmenge von 0,4 bis 0,9 Gew.-%, vorzugsweise von 0,5 bis 0,8 Gew.-%, insbesondere von 0,6 bis 0,8 Gew.-%, enthält.
7. Kosmetisches Mittel nach einem der vorangehenden Punkte, dadurch gekennzeichnet, dass in der Formel (I) R für eine lineare oder verzweigte Alkylkette mit 12 bis 14 Kohlenstoffatomen, insbesondere mit 13 Kohlenstoffatomen, und n für ganze Zahlen von 7 bis 12, vorzugsweise von 8 bis 11, bevorzugt von 9 oder 10, insbesondere von 10, steht.
8. Kosmetisches Mittel nach einem der vorangehenden Punkte, dadurch gekennzeichnet, dass das kosmetische Mittel das mindestens eine nichtionische Tensid c) der Formel (I) in einer Gesamtmenge von 0,0005 bis 10 Gew.-%, vorzugsweise von 0,001 bis 8,0 Gew.-%, bevorzugt von 0,005 bis 5,0 Gew.-%, insbesondere von 0,01 bis 1,0 Gew.-%, enthält.
9. Kosmetisches Mittel nach einem der vorangehenden Punkte, dadurch gekennzeichnet, dass das kosmetische Mittel zusätzlich mindestens ein Dimethylcyclosiloxan in einer Gesamtmenge von 0,001 bis 2,0 Gew.-%, vorzugsweise von 0,05 bis 1,5 Gew.-%, insbesondere von 0,01 bis 1,0 Gew.-%, bezogen auf das Gesamtgewicht des kosmetischen Mittels, enthält, wobei das mindestens eine Dimethylcyclosiloxan die Formel (II) aufweist

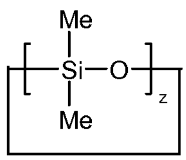
(II),

worin
z für ganze Zahlen von 2 bis 8, vorzugsweise von 2 bis 6, bevorzugt von 2 bis 4, insbesondere für die ganze Zahl 4, steht.
10. Kosmetisches Mittel nach einem der vorangehenden Punkte, dadurch gekennzeichnet, dass das kosmetische Mittel zusätzlich mindestens eine weitere Verbindung, ausgewählt aus der Gruppe von Verdickungsmitteln, linearen oder verzweigten, gesättigten oder ungesättigten Alkoholen mit 8 bis 20 Kohlenstoffatomen, Alkalisierungsmitteln sowie deren Mischungen, enthält.
11. Kosmetisches Mittel nach Punkt 10, dadurch gekennzeichnet, dass als Verdickungsmittel mindestens ein natürlich vorkommendes Verdickungsmittel, insbesondere Xanthangum sowie dessen Salze, in einer Gesamtmenge von 0,0005 bis 5,0 Gew.-%, vorzugsweise von 0,001 bis 1,0 Gew.-%, bevorzugt von 0,005 bis 0,5 Gew.-%, insbesondere von 0,01 bis 0,2 Gew.-%, bezogen auf das Gesamtgewicht des kosmetischen Mittels, enthalten ist.
12. Verpackungseinheit (Kit-of-Parts), umfassend - getrennt voneinander konfektioniert -

a) mindestens einen Container (C1), enthaltend ein kosmetisches Mittel (M1) nach einem der Punkte 1 bis 11, und

b) mindestens einen Container (C2), enthaltend eine Oxidationsmittelzubereitung (M2), welche in einem kosmetisch verträglichen Träger mindestens ein Oxidationsmittel sowie mindestens eine Säure enthält.

13. Verfahren zum Färben von keratinischen Fasern, wobei das Verfahren die folgenden Verfahrensschritte umfasst:

a) Bereitstellen eines kosmetischen Mittels (M1) nach einem der Punkte 1 bis 11,
b) Bereitstellen einer Oxidationsmittelzubereitung (M2), enthaltend in einem kosmetisch verträglichen Träger mindestens ein Oxidationsmittel sowie mindestens eine Säure,
c) Vermischen des kosmetischen Mittels (M1) mit der Oxidationsmittelzubereitung (M2),
d) Applizieren der in Schritt c) erhaltenen Mischung auf die keratinischen Fasern und Belassen dieser Mischung für eine Zeit von 10 bis 60 Minuten, bevorzugt von 20 bis 45 Minuten, bei Raumtemperatur und/oder bei mindestens 30 °C auf den keratinischen Fasern,
e) Spülen der keratinischen Fasern mit Wasser und/oder einer reinigenden Zusammensetzung für 1 bis 5 Minuten, und
f) gegebenenfalls Applizieren eines Nachbehandlungsmittels auf die keratinischen Fasern und Ausspülen nach einer Zeit von 1 bis 10 Minuten.

14. Verwendung eines kosmetischen Mittels nach einem der Punkte 1 bis 11 zur Erhöhung der Farbtiefe.
15. Verwendung einer Verpackungseinheit (Kit-of-parts) nach Punkt 12 zur Herstellung eines kosmetischen Mittels zur Farbveränderung keratinischer Fasern mit erhöhter Farbtiefe.

Die nachfolgenden Beispiele sollen bevorzugte Ausführungsformen der Erfindung erläutern, ohne sie jedoch einzuschränken.

Beispiele:

1. Rezepturen

Zusammensetzungen der eingesetzten kosmetischen Mittel (Öl-in-Wasser-Emulsionen, alle Mengen in Gew.-%). Das in den nachfolgenden Formulierungen verwendete nichtionische Tensid ist bevorzugt ein nichtionisches Tensid der Formel (I), worin R für eine lineare Alkylkette mit 13 Kohlenstoffatomen und n für die ganze Zahl 10 steht.

| Rohstoff | V1 | V2 | E1* |
|---|---|---|---|
| Xanthan Gum | 0,1 | 0,1 | 0,1 |
| 2-Octyldodecanol | 2,3 | 2,3 | 2,3 |
| Lanette N [a)] | 14 | 14 | 14 |
| Cetearyl Alkohol | 3,9 | 3,9 | 3,9 |
| Cutina GMS-SE [b)] | 6,0 | 6,0 | 6,0 |
| Glycerol 99,5 % | 2,0 | 2,0 | 2,0 |
| Kokosamidopropylbeatin, 40%ig | 2,0 | 2,0 | 2,0 |
| Monoethanolamin | 4,3 | 4,3 | 4,3 |
| 2-Amino-2-methylpropanol | 0,1 | 0,1 | 0,1 |
| Natriumsulfit, wasserfrei | 0,2 | 0,2 | 0,2 |
| Caramelsirup, 75%ig | 0,1 | 0,1 | 0,1 |
| Traubenkernöl | 1,0 | 1,0 | 1,0 |
| p-Toluylendiaminsulfat | 0,9 | 0,9 | 0,9 |
| Resorcinol | 0,3 | 0,3 | 0,3 |
| 3-Aminophenol | 0,1 | 0,1 | 0,1 |
| 4-Chlorresorcinol | 0,2 | 0,2 | 0,2 |
| Vernetztes, aminiertes Siliconpolymer ** | - | 1,4 | 0,7 |

(fortgesetzt)

| Rohstoff | V1 | V2 | E1* |
|---|---|---|---|
| Nichtionisches Tensid der Formel (I) | - | 0,1 | 0,05 |
| Wasser, vollentsalzt | ad 100,00 | ad 100,00 | ad 100,00 |
| * erfindungsgemäß<br>** Aktivsubstanz<br>a) INCI-Bezeichnung: Cetearyl alcohol, Sodium cetearyl sulfate (BASF)<br>b) INCI-Bezeichnung: Glyceryl stearate (BASF) | | | |

Die Fettbasis wurde jeweils zusammen bei 80°C aufgeschmolzen und mit einem Teil der Wassermenge dispergiert. Anschließend wurden die restlichen Rezepturbestandteile unter Rühren der Reihe nach eingearbeitet. Dann wurde mit Wasser auf 100 Gew.-% aufgefüllt und die Formulierung kalt gerührt. Bei den Rezepturen V1 und V2 handelt es sich um nicht erfindungsgemäße Vergleichsrezepturen ohne aminiertes Siliconpolymer/nichtionisches Tensid bzw. mit einer zu hohen Menge an vernetztem, aminiertem Siloxanpolymer. Die Rezeptur E1 ist ein erfindungsgemäßes Beispiel.

Oxidationsmittelzubereitung O1 (alle Mengen in Gew.-%)

| Rohstoff | O1 |
|---|---|
| Dinatriumpyrophosphat | 0,10 |
| Dipicolinsäure | 0,10 |
| Kaliumhydroxid 50% | 0,30 |
| 1-Hydroxyethan-1,1-diphosphonsäure 60% | 0,40 |
| Emulgade F c) | 4,0 |
| Cetearyl Alcohol | 0,5 |
| Ceteareth-20 | 0,5 |
| Bienenwachs | 0,3 |
| Isopropylmyristat | 10 |
| Wasserstoffperoxid 50 % | 11 |
| Wasser vollentsalzt | ad 100 |
| c) INCI-Bezeichnung: Cetearyl alcohol, PEG-40 Castor oil, Sodium cetearyl sulfat (BASF) | |

2. Erhöhung der Farbtiefe

Zur Herstellung der oxidativen Färbemittel für die Bestimmung der Farbtiefe wurden die kosmetischen Mittel V1, V2 sowie E2 jeweils im Gewichtsverhältnis 1:1 mit der obigen Oxidationsmittelzubereitung O1 vermischt.

Die auf diese Weise hergestellten oxidativen Färbemittel wurden jeweils in definierter Menge (4 g oxidatives Färbemittel pro 1 g Yakhaar) auf Yakhaar-Strähnen aufgetragen (jeweils 12 Strähnen pro oxidativem Färbemittel) und verblieben für eine Einwirkdauer von 30 Minuten bei 32 °C auf den Haarsträhnen. Anschließend wurden die verbliebenen Mittel jeweils 2 Minuten lang mit lauwarmem Wasser aus den Haarsträhnen ausgespült, die Strähnen zunächst mit einem Handtuch getrocknet und anschließend trocken geföhnt.

Alle Strähnen wurden mit einem Farbmessgerät der Firma Datacolor, Typ Spectraflash 450, vermessen. Der für die Beurteilung der Farbtiefe herangezogene Wert dL ergibt sich aus den an der jeweiligen Strähne gemessenen L -Farbmesswerten wie folgt:

$$dL = L_i - L_0$$

Lo sind hierbei die Mittelwerte der aus den 12 Messungen ermittelten Farbmesswerte der mit dem oxidativen Färbemittel V1 behandelten Yakhaar-Strähnen, während $L_i$ die Mittelwerte der Farbmesswerte nach der oxidativen Färbung der Haarsträhnen mit den oxidativen Färbemitteln V2 und E1 darstellen.

Je geringer der dL-Wert ist, desto höher ist die Farbtiefe. In der nachfolgenden Tabelle sind die dL-Werte für die Ausfärbungen unter Verwendung der kosmetischen Mittel V2 und E1 dargestellt. Die Ausfärbungen mit dem erfindungsgemäßen kosmetischen Mittel E2, welche das spezielles vernetzte, aminierte Siloxanpolymer in einer Gesamtmenge von 0,7 Gew.-% sowie ein spezielles nichtionisches Tensid enthalten, weisen eine erhöhte Farbtiefe gegenüber dem Färbemittel V1 ohne vernetztem, aminiertem Siloxanpolymer/nichtionischem Tensid auf. Dahingegen zeigen die Ausfärbungen mit dem nicht erfindungsgemäßen kosmetischen Mittel V2, welches das vernetzte, aminierte Siloxanpolymer in einer Gesamtmenge von mehr als 1 Gew.-%, nämlich in 1,4 Gew.-%, enthält, eine verringerte Farbtiefe gegenüber den Färbemitteln V1 ohne vernetztem, aminiertem Siloxanpolymer/nichtionischem Tensid.

| Oxidatives Färbemittel | dL |
|---|---|
| V2 + O1 (1:1) | -0,84 |
| E1 + O1 (1:1) | +2,76 |

Neben dem dL-Wert kann weiterhin die relative Farbstärke herangezogen werden, um eine Erhöhung bzw. Erniedrigung der Farbtiefe zu bestimmten. Die relative Farbstärke wird anhand der Helligkeitswerte L wie folgt bestimmt

$$\text{relative Farbstärke [\%]} = (L_0/L_i)*100$$

Lo sind hierbei die Mittelwerte der aus den 12 Messungen ermittelten Farbmesswerte der mit dem oxidativen Färbemittel V1 behandelten Yakhaar-Strähnen, während $L_i$ die Mittelwerte der Farbmesswerte nach der oxidativen Färbung der Haarsträhnen mit den oxidativen Färbemitteln V2 und E1 darstellen.

Ein Wert von 100 % für die relative Farbstärke bedeutet hierbei, dass die mit den Färbemitteln V2 und E1 erzielten Ausfärbungen eine identische Helligkeit besitzen wie die mit dem Färbemittel V1 erzielte Ausfärbung. Die Farbtiefe der Färbemittel V2 und E1 ist in diesem Fall identisch mit der Farbtiefe des Färbemittels V1. Werte über 100 % bedeuten eine erhöhte Farbtiefe gegenüber der Vergleichszusammensetzung V1, wohingegen Werte unter 100 % für eine verringerte Farbtiefe gegenüber V1 stehen. Die relativen Farbstärken der Färbemittel V2 und E1 sind in nachfolgender Tabelle zusammengefasst. Das erfindungsgemäße Färbemittel E1 weist eine relative Farbstärke von mehr als 100 %, d. h. eine höhere Farbtiefe als die Vergleichszusammensetzung V1 ohne vernetztem, aminiertem Siloxanpolymer/nichtionischem Tensid, auf. Dahingegen weist das nicht erfindungsgemäße Färbemittel V2 mit einem Gesamtgewicht von vernetztem, aminiertem Siloxanpolymer von mehr als 1 Gew.-% eine relative Farbstärke von weniger als 100 % und somit eine gegenüber der Vergleichszusammensetzung V1 ohne vernetztem, aminiertem Siloxanpolymer/nichtionischem Tensid verringerte Farbtiefe auf.

| Oxidatives Färbemittel | Relative Farbstärke [%] |
|---|---|
| V2 + O1 (1:1) | 104 |
| E1 + O1 (1:1) | 81,3 |

**Patentansprüche**

**1.** Kosmetisches Mittel zur Farbveränderung keratinischer Fasern, enthaltend in einem kosmetisch verträglichen Träger

a) mindestens eine Verbindung, ausgewählt aus der Gruppe von Oxidationsfarbstoffvorprodukten, direktziehenden Farbstoffen sowie deren Mischungen,
b) mindestens ein vernetztes, aminiertes Siloxanpolymer, welches durch Umsetzung eines Copolymers, bestehend aus 3-(2-Aminoethylamino)propylmethylsiloxyeinheiten und Dimethylsiloxyeinheiten, mit N-Morpholinomethyltriethoxysilan erhältlich ist, in einer Gesamtmenge von 0,3 bis 1,0 Gew.-%, bezogen auf das Gesamtgewicht des kosmetischen Mittels, und
c) mindestens ein nichtionisches Tensid der Formel (I)

$$R\text{-}O\text{-}(CH_2\text{-}CH_2\text{-}O)_n\text{-}H \qquad (I),$$

worin

R für eine lineare oder verzweigte Alkylkette mit 12 bis 16 Kohlenstoffatomen steht, und
n für ganze Zahlen von 6 bis 15 steht.

**2.** Kosmetisches Mittel nach Anspruch 1, **dadurch gekennzeichnet, dass** das mindestens eine vernetzte, aminierte Siloxanpolymer b) als Emulsion vorliegt, wobei die Emulsion eine mittlere Teilchengröße Dso von 3 bis 500 nm, vorzugsweise von 10 bis 400 nm, bevorzugt von 50 bis 300 nm, insbesondere von 100 bis 300 nm, aufweist.

**3.** Kosmetisches Mittel nach einem der Ansprüche 1 oder 2, **dadurch gekennzeichnet, dass** das kosmetische Mittel das mindestens eine vernetzte, aminierte Siloxanpolymer b) in einer Gesamtmenge von 0,4 bis 0,9 Gew.-%, vorzugsweise von 0,5 bis 0,8 Gew.-%, insbesondere von 0,6 bis 0,8 Gew.-%, enthält.

**4.** Kosmetisches Mittel nach einem der vorangehenden Ansprüche, **dadurch gekennzeichnet, dass** in der Formel (I) R für eine lineare oder verzweigte Alkylkette mit 12 bis 14 Kohlenstoffatomen, insbesondere mit 13 Kohlenstoffatomen, und n für ganze Zahlen von 7 bis 12, vorzugsweise von 8 bis 11, bevorzugt von 9 oder 10, insbesondere von 10, steht.

**5.** Kosmetisches Mittel nach einem der vorangehenden Ansprüche, **dadurch gekennzeichnet, dass** das kosmetische Mittel das mindestens eine nichtionische Tensid c) der Formel (I) in einer Gesamtmenge von 0,0005 bis 10 Gew.-%, vorzugsweise von 0,001 bis 8,0 Gew.-%, bevorzugt von 0,005 bis 5,0 Gew.-%, insbesondere von 0,01 bis 1,0 Gew.-%, enthält.

**6.** Kosmetisches Mittel nach einem der vorangehenden Ansprüche, **dadurch gekennzeichnet, dass** das kosmetische Mittel zusätzlich mindestens ein Dimethylcyclosiloxan in einer Gesamtmenge von 0,001 bis 2,0 Gew.-%, vorzugsweise von 0,05 bis 1,5 Gew.-%, insbesondere von 0,01 bis 1,0 Gew.-%, bezogen auf das Gesamtgewicht des kosmetischen Mittels, enthält, wobei das mindestens eine Dimethylcyclosiloxan die Formel (II) aufweist

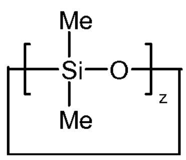

(II),

worin
z für ganze Zahlen von 2 bis 8, vorzugsweise von 2 bis 6, bevorzugt von 2 bis 4, insbesondere für die ganze Zahl 4, steht.

**7.** Verpackungseinheit (Kit-of-Parts), umfassend - getrennt voneinander konfektioniert -

a) mindestens einen Container (C1), enthaltend ein kosmetisches Mittel (M1) nach einem der Ansprüche 1 bis 6, und
b) mindestens einen Container (C2), enthaltend eine Oxidationsmittelzubereitung (M2), welche in einem kosmetisch verträglichen Träger mindestens ein Oxidationsmittel sowie mindestens eine Säure enthält.

**8.** Verfahren zum Färben von keratinischen Fasern, wobei das Verfahren die folgenden Verfahrensschritte umfasst:

a) Bereitstellen eines kosmetischen Mittels (M1) nach einem der Ansprüche 1 bis 6,
b) Bereitstellen einer Oxidationsmittelzubereitung (M2), enthaltend in einem kosmetisch verträglichen Träger mindestens ein Oxidationsmittel sowie mindestens eine Säure,
c) Vermischen des kosmetischen Mittels (M1) mit der Oxidationsmittelzubereitung (M2),
d) Applizieren der in Schritt c) erhaltenen Mischung auf die keratinischen Fasern und Belassen dieser Mischung für eine Zeit von 10 bis 60 Minuten, bevorzugt von 20 bis 45 Minuten, bei Raumtemperatur und/oder bei mindestens 30 °C auf den keratinischen Fasern,
e) Spülen der keratinischen Fasern mit Wasser und/oder einer reinigenden Zusammensetzung für 1 bis 5

Minuten, und
f) gegebenenfalls Applizieren eines Nachbehandlungsmittels auf die keratinischen Fasern und Ausspülen nach einer Zeit von 1 bis 10 Minuten.

**9.** Verwendung eines kosmetischen Mittels nach einem der Ansprüche 1 bis 6 zur Erhöhung der Farbtiefe.

**10.** Verwendung einer Verpackungseinheit (Kit-of-parts) nach Anspruch 7 zur Herstellung eines kosmetischen Mittels zur Farbveränderung keratinischer Fasern mit erhöhter Farbtiefe.

## Claims

**1.** A cosmetic agent for changing the color of keratinic fibers, containing, in a cosmetically acceptable carrier,

a) at least one compound, selected from the group of oxidation dye precursors, direct dyes, and mixtures thereof,
b) at least one crosslinked, aminated siloxane polymer that is obtainable by reacting a copolymer, consisting of 3-(2-aminoethylamino)propylmethylsiloxy units and dimethylsiloxy units, with N-morpholinomethyltriethoxysilane, in a total amount of 0.3 to 1.0% by weight, based on the total weight of the cosmetic agent, and
c) at least one nonionic surfactant of the formula (I)

$$R\text{-}O\text{-}(CH_2\text{-}CH_2\text{-}O)_n\text{-}H \qquad (I),$$

where

R stands for a linear or branched alkyl chain having 12 to 16 carbon atoms, and
n stands for integers from 6 to 15.

**2.** The cosmetic agent according to claim 1, **characterized in that** the at least one crosslinked, aminated siloxane polymer b) is present as an emulsion, wherein the emulsion has an average particle size $D_{50}$ of 3 to 500 nm, primarily of 10 to 400 nm, preferably of 50 to 300 nm, in particular of 100 to 300 nm.

**3.** The cosmetic agent according to one of claims 1 or 2, **characterized in that** the cosmetic agent contains the at least one crosslinked, aminated siloxane polymer b) in a total amount of 0.4 to 0.9% by weight, preferably of 0.5 to 0.8% by weight, in particular of 0.6 to 0.8% by weight.

**4.** The cosmetic agent according to one of the preceding claims, **characterized in that** in the formula (I) R stands for a linear or branched alkyl chain having 12 to 14 carbon atoms, in particular having 13 carbon atoms, and n for integers from 7 to 12, primarily from 8 to 11, preferably from 9 or 10, in particular 10.

**5.** The cosmetic agent according to one of the preceding claims, **characterized in that** the cosmetic agent contains the at least one nonionic surfactant c) of the formula (I) in a total amount of 0.0005 to 10% by weight, primarily of 0.001 to 8.0% by weight, preferably of 0.005 to 5.0% by weight, in particular of 0.01 to 1.0% by weight.

**6.** The cosmetic agent according to one of the preceding claims, **characterized in that** the cosmetic agent contains in addition at least one dimethylcyclosiloxane in a total amount of 0.001 to 2.0% by weight, preferably of 0.05 to 1.5% by weight, in particular of 0.01 to 1.0% by weight, based on the total weight of the cosmetic agent, the at least one dimethylcyclosiloxane having the formula (II)

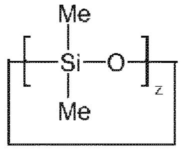 (II),

where
z stands for integers from 2 to 8, primarily from 2 to 6, preferably from 2 to 4, in particular for the integer 4.

7. A packaging unit (kit of parts), comprising, produced separately from one another,

a) at least one container (C1), containing a cosmetic agent (M1) according to one of claims 1 to 6, and
b) at least one container (C2), containing an oxidizing agent preparation (M2), which contains at least one oxidizing agent and at least one acid in a cosmetically acceptable carrier.

8. A method for dyeing keratinic fibers, wherein the method comprises the following process steps:

a) providing a cosmetic agent (M1) according to one of claims 1 to 6,
b) providing an oxidizing agent preparation (M2), containing in a cosmetically acceptable carrier at least one oxidizing agent and at least one acid,
c) mixing the cosmetic agent (M1) with the oxidizing agent preparation (M2),
d) applying the mixture obtained in step c) to the keratinic fibers and leaving said mixture on the keratinic fibers for a time period of 10 to 60 minutes, preferably of 20 to 45 minutes, at room temperature and/or at at least 30°C,
e) rinsing the keratinic fibers with water and/or a cleansing composition for 1 to 5 minutes, and
f) optionally applying an aftertreatment agent to the keratinic fibers and rinsing it off after a time period of 1 to 10 minutes.

9. Use of a cosmetic agent according to one of claims 1 to 6 for increasing the color depth.

10. Use of a packaging unit (kit of parts) according to claim 7 for producing a cosmetic agent for changing the color of keratinic fibers with an increased color depth.

## Revendications

1. Agent cosmétique conçu pour modifier la couleur des fibres kératiniques contenant, dans un support cosmétiquement acceptable,

a) au moins un composé sélectionné dans le groupe constitué par les précurseurs de colorant par oxydation, les colorants directs ainsi que les mélanges de ceux-ci,
b) au moins un polymère de siloxane aminé réticulé obtenu par réaction d'un copolymère constitué de motifs 3-(2-aminoéthylamino)propylméthylsiloxy et de motifs diméthylsiloxy avec du N-morpholinométhyltriéthoxysilane, en une quantité totale située dans la plage allant de 0,3 à 1,0 % en poids, par rapport au poids total de l'agent cosmétique, et
c) au moins un tensioactif non ionique de formule (I)

$$R\text{-}O\text{-}(CH_2\text{-}CH_2\text{-}O)_n\text{-}H \qquad (I),$$

dans laquelle
R représente une chaîne alkyle linéaire ou ramifiée de 12 à 16 atomes de carbone et n représente des nombres entiers allant de 6 à 15.

2. Agent cosmétique selon la revendication 1, **caractérisé en ce que** l'au moins un polymère de siloxane aminé réticulé b) se présente sous la forme d'une émulsion, l'émulsion présentant une taille moyenne de particule $D_{50}$ située dans la plage allant de 3 à 500 nm, de préférence de 10 à 400 nm, de manière préférée de 50 à 300 nm, en particulier de 100 à 300 nm.

3. Agent cosmétique selon l'une des revendications 1 ou 2, **caractérisé en ce que** l'agent cosmétique contient l'au moins un polymère de siloxane aminé réticulé b) en une quantité totale située dans la plage allant de 0,4 à 0,9 % en poids, de préférence de 0,5 à 0,8 % en poids, en particulier de 0,6 à 0,8 % en poids.

4. Agent cosmétique selon l'une des revendications précédentes, **caractérisé en ce que**, dans la formule (I), R représente une chaîne alkyle linéaire ou ramifiée de 12 à 14 atomes de carbone, en particulier de 13 atomes de carbone, et n représente des nombres entiers allant de 7 à 12, de préférence de 8 à 11, de manière davantage préférée 9 ou 10, en particulier 10.

5. Agent cosmétique selon l'une des revendications précédentes, **caractérisé en ce que** l'agent cosmétique contient

l'au moins un tensioactif non ionique c) de formule (I) en une quantité totale située dans la plage allant de 0,0005 à 10 % en poids, de préférence de 0,001 à 8,0 % en poids, de manière davantage préférée de 0,005 à 5,0 % en poids, en particulier de 0,01 à 1,0 % en poids.

**6.** Agent cosmétique selon l'une des revendications précédentes, **caractérisé en ce que** l'agent cosmétique contient en outre au moins un diméthylcyclosiloxane en une quantité totale située dans la plage allant de 0,001 à 2,0 % en poids, de préférence de 0,05 à 1,5 % en poids, en particulier de 0,01 à 1,0 % en poids, par rapport au poids total de l'agent cosmétique, l'au moins un diméthylcyclosiloxane répondant à la formule (II),

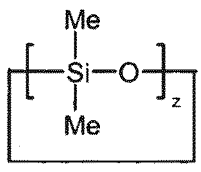 (II),

dans laquelle
z représente des nombres entiers allant de 2 à 8, de préférence de 2 à 6, de manière davantage préférée de 2 à 4, en particulier le nombre entier 4.

**7.** Unité de conditionnement (*kit*) comprenant - conditionnés séparément l'un de l'autre :

a) au moins un conteneur (C1) contenant un agent cosmétique (M1) selon l'une des revendications 1 à 6, et
b) au moins un conteneur (C2) contenant une préparation d'agent oxydant (M2) qui contient, dans un support cosmétiquement acceptable, au moins un agent oxydant et au moins un acide.

**8.** Procédé de coloration des fibres kératiniques, le procédé comprenant les étapes de procédé suivantes :

a) la fourniture d'un agent cosmétique (M1) selon l'une des revendications 1 à 6,
b) la fourniture d'une préparation d'agent oxydant (M2) contenant, dans un support cosmétiquement acceptable, au moins un agent oxydant et au moins un acide,
c) le mélange de l'agent cosmétique (M1) avec la préparation d'agent d'oxydation (M2),
d) l'application sur les fibres kératiniques du mélange obtenu à l'étape c) et la pose de ce mélange sur les fibres kératiniques pendant une durée située dans la plage allant de 10 à 60 minutes, de préférence de 20 à 45 minutes, à la température ambiante et/ou à au moins 30°C,
e) le rinçage des fibres kératiniques avec de l'eau et/ou une composition détergente pendant 1 à 5 minutes, et
f) éventuellement, l'application d'un agent de traitement complémentaire sur les fibres kératiniques et l'élimination de celui-ci par rinçage après une durée située dans la plage allant de 1 à 10 minutes.

**9.** Utilisation d'un agent cosmétique selon l'une des revendications 1 à 6 pour intensifier la profondeur de couleur.

**10.** Utilisation d'une unité de conditionnement (*kit*) selon la revendication 7 pour préparer un agent cosmétique conçu pour modifier la couleur des fibres kératiniques par intensification de la profondeur de couleur.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **LIU X. M.** Comparative Studies of Poly(DimethylSiloxanes) Using Automated GPC-MALDI-TOF MS and On-Line GPC-ESI-TOF MS. *Am. Soc. Mass. Spectrom.,* 2003, vol. 14, 195-202
- Collodial Silica-Fundamentals and Application. **BERGNA H. E.** Surfactant science series. CRC Press, 2006, vol. 131, 65-80